(19) Europäisches Patentamt
European Patent Office
Office européen des brevets

(11) Veröffentlichungsnummer : **0 126 355**
**B1**

(12) # EUROPÄISCHE PATENTSCHRIFT

(45) Veröffentlichungstag der Patentschrift : **09.09.87**

(21) Anmeldenummer : **84105080.0**

(22) Anmeldetag : **05.05.84**

(51) Int. Cl.[4] : **C 07 D215/56**, C 07 D401/04, C 07 D413/04, C 07 D417/04, A 61 K 31/47, A 61 K 31/495, A 61 K 31/535, A 61 K 31/54

(54) **7-Amino-1-cyclopropyl-6,8-difluor-1,4-dihydro-4-oxo-3-chinolincarbonsäuren, Verfahren zu ihrer Herstellung sowie diese enthaltende antibakterielle Mittel.**

(30) Priorität : **18.05.83 DE 3318145**

(43) Veröffentlichungstag der Anmeldung : **28.11.84 Patentblatt 84/48**

(45) Bekanntmachung des Hinweises auf die Patenterteilung : **09.09.87 Patentblatt 87/37**

(84) Benannte Vertragsstaaten : **AT BE CH DE FR GB IT LI LU NL SE**

(56) Entgegenhaltungen :
**EP-A- 0 004 279**
**EP-A- 0 028 698**
**EP-A- 0 049 355**
**EP-A- 0 049 593**
**EP-A- 0 067 666**
**EP-A- 0 078 362**
**DE-A- 2 840 910**
**DE-A- 3 106 013**
**DE-A- 3 205 655**
**Die Akte enthält technische Angaben, die nach dem Eingang der Anmeldung eingereicht wurden und die nicht in dieser Patentschrift enthalten sind.**

(73) Patentinhaber : **BAYER AG**
**Konzernverwaltung RP Patentabteilung**
**D-5090 Leverkusen 1 Bayerwerk (DE)**

(72) Erfinder : **Grohe, Klaus, Dr.**
**Am Wasserturm 10**
**D-5068 Odenthal (DE)**
Erfinder : **Petersen, Uwe, Dr.**
**Auf dem Forst 4**
**D-5090 Leverkusen 1 (DE)**
Erfinder : **Zeiler, Hans-Joachim, Dr.**
**Elsbeekerstrasse 46**
**D-5620 Velbert 15 (DE)**
Erfinder : **Metzger, Karl Georg, Dr.**
**Pahlkestrasse 75**
**D-5600 Wuppertal 1 (DE)**

**EP 0 126 355 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft neue 7-Amino-1-cyclopropyl-6,8-difluor-1,4-dihydro-4-oxo-3-chinolincarbonsäuren, Verfahren zu ihrer Herstellung sowie diese enthaltende antibakterielle Mittel.

Gefunden wurden die neuen 7-Amino-1-cyclopropyl-6,8-difluor-1,4-dihydro-4-oxo-3-chinolincarbonsäuren der Formel (I)

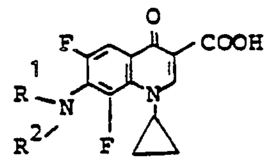

(I)

in welcher

$R^1$ und $R^2$ gleich oder verschieden sein können und für einen gegebenenfalls durch eine Hydroxy-, Amino-, Methylamino- oder Dimethylaminogruppe substituierten $C_1$-$C_4$-Alkylrest stehen und weiterhin gemeinsam mit dem Stickstoffatom, an das sie gebunden sind, einen 5- oder 6-gliedrigen heterocyclischen Ring bilden können, der als Ringglied zusätzlich die Atome oder Gruppen —O—, —S—, —SO—, —$SO_2$— oder >N—$R^3$ enthalten kann und der gegebenenfalls an den Kohlenstoffatomen ein- bis dreifach durch $C_1$-$C_4$-Alkyl, Hydroxy, Alkoxy mit 1-3 Kohlenstoffatomen, Amino, Methylamino oder Ethylamino substituiert sein kann, wobei jeweils ein Kohlenstoffatom nur einen Substituenten tragen kann, wobei

$R^3$ für Wasserstoff, eine verzweigte oder unverzweigte Alkyl-, Alkenyl- oder Alkinylgruppe mit 1 bis 6 Kohlenstoffatomen, die gegebenenfalls durch eine Hydroxy-, Trifluormethylmercapto-, Alkoxy-, Alkylmercapto-, Alkylamino- oder Dialkylaminogruppe mit 1 bis 3 Kohlenstoffatomen für einen Alkylrest, die Cyanogruppe, Alkoxycarbonylgruppe mit 1 bis 4 Kohlenstoffatomen im Alkoholteil substituiert sein kann, die Benzyloxycarbonylgruppe, eine gegebenenfalls im Phenylrest durch Nitro oder Amino substituierte Phenylalkylgruppe mit bis zu 4 Kohlenstoffatomen im aliphatischen Teil, einen gegebenenfalls durch Hydroxy, Methoxy, Chlor und Fluor ein- oder zweifach substituierten Phenylrest, einen gegebenenfalls durch Hydroxy, Methoxy, Chlor und Fluor ein- oder zweifach substituierten Phenacylrest, einen Oxoalkylrest mit bis zu 6 Kohlenstoffatomen sowie einen Cycloalkyl-alkylrest mit bis zu 6 Kohlenstoffatomen im cyclischen Teil und bis zu 3 Kohlenstoffatomen im acyclischen Teil steht, ferner einen Rest $COR^4$, CN oder $SO_2R^5$ bedeutet, wobei

$R^4$ Wasserstoff, gegebenenfalls durch 1 oder 2 Substituenten aus der Reihe Amino, Alkoxycarbonyl mit 1 bis 3 Kohlenstoffatomen im Alkylteil, Carboxy, Alkoxy mit 1 bis 3 Kohlenstoffatomen oder Trifluormethylthio substituiertes geradkettiges oder verzweigtes Alkyl mit 1 bis 4 C-Atomen, gegebenenfalls durch Chlor, Hydroxy, Amino oder Carboxy substituiertes Phenyl, Alkoxy mit 1 bis 4 C-Atomen, Alkylthio mit 1 bis 2 C-Atomen, Benzyloxy, Amino, gegebenenfalls durch Alkoxycarbonyl mit 1 bis 3 C-Atomen im Alkylteil oder Carboxy substituiertes Alkylamino mit 1 bis 5 C-Atomen,

$R^5$ geradkettiges oder verzweigtes Alkyl mit 1 bis 3 C-Atomen, Phenyl oder Methylphenyl darstellt, und deren pharmazeutisch verwendbaren Salze.

Aus der DE-A1-3 106 013 und der EP-A3-0 078 362 sind antibakteriell wirksame Chinoloncarbonsäuren bekannt. Die erfindungsgemäßen Verbindungen zeigen demgegenüber keimspezifisch verbesserte antibakterielle Wirksamkeiten.

Sie eignen sich daher als Wirkstoffe für die Human- und Veterinärmedizin, wobei zur Veterinärmedizin auch die Vorbeugung und Behandlung bei Fischen zu zählen ist.

Bevorzugt sind diejenigen Verbindungen der Formel (I), in denen

$R^1$ und $R^2$ gleich oder verschieden sein können und für einen gegebenenfalls durch eine Hydroxy- oder Aminogruppe substituierten $C_1$-$C_3$-Alkylrest stehen und weiterhin gemeinsam mit dem Stickstoffatom, an das sie gebunden sind, einen 5- oder 6-gliedrigen heterocyclischen Ring bilden können, der als Ringglied zusätzlich die Atome der Gruppen —O—, —S—, —$SO_2$— oder >N—$R^3$ enthalten kann und der gegebenenfalls an den Kohlenstoffatomen ein- bis zweifach durch $C_1$-$C_3$-Alkyl, Hydroxy, Amino oder Methylamino substituiert sein kann, wobei jeweils ein Kohlenstoffatom nur einen Substituenten tragen kann, wobei

$R^3$ für Wasserstoff, eine verzweigte oder unverzweigte Alkyl-, Alkenyl- oder Alkinylgruppe mit 1 bis 4 Kohlenstoffatomen, die gegebenenfalls durch eine Hydroxy-, Trifluormethylmercapto-, Alkoxy- oder Alkylmercaptogruppe mit 1 bis 2 Kohlenstoffatomen für einen Alkylrest, die Cyanogruppe, die Alkoxycarbonylgruppe mit 1 bis 3 Kohlenstoffatomen im Alkoholteil substituiert sein kann, die Benzyloxycarbonylgruppe, eine gegebenenfalls im Phenylrest durch Nitro oder Amino substituierte Phenyalkylgruppe mit bis zu 2 Kohlenstoffatomen im aliphatischen Teil, einen Phenacylrest, einen Oxoalkylrest mit bis zu 5 Kohlenstoffatomen sowie einen Cycloalkyl-alkylrest mit bis zu 6 Kohlenstoffatomen im cyclischen Teil und bis zu 2 Kohlenstoffatomen im acyclischen Teil steht, ferner einen Rest $COR^4$, CN oder $SO_2R^5$ bedeutet, wobei

$R^4$ Wasserstoff, gegebenenfalls durch 1 oder 2 Substituenten aus der Reihe Amino, Methoxycarbonyl,

Carboxy, Alkoxy mit 1 bis 2 Kohlenstoffatomen oder Trifluormethylthio substituiertes geradkettiges oder verzweigtes Alkyl mit 1 bis 3 C-Atomen, gegebenenfalls durch Chlor oder Hydroxy substituiertes Phenyl, Alkoxy mit 1 bis 3 C-Atomen, Amino, gegebenenfalls durch Alkoxycarbonyl mit 1 bis 2 C-Atomen im Alkylteil oder Carboxy substituiertes Alkylamino mit 1 bis 5 C-Atomen und

$R^5$ Methyl, Ethyl, Phenyl oder Methylphenyl bedeutet.

Besonders bevorzugt sind diejenigen Verbindungen der Formel (I), in denen

$R^1$ und $R^2$ gleich oder verschieden sein können und für einen gegebenenfalls durch eine Hydroxygruppe substituierten $C_1$-$C_2$-Alkylrest stehen und weiterhin gemeinsam mit dem Stickstoffatom, an das sie gebunden sind, einen 6-gliedrigen heterocyclischen Ring bilden können, der als Ringglied zusätzlich die Gruppe N—$R^3$ enthalten kann und der gegebenenfalls an den Kohlenstoffatomen ein- bis zweifach durch $C_1$-$C_2$-Alkyl oder Hydroxy substituiert sein kann, wobei, jeweils ein Kohlenstoffatom nur einen Substituenten tragen kann, wobei

$R^3$ für Wasserstoff, eine verzweigte oder unverzweigte Alkyl-, Alkenyl- oder Alkinylgruppe mit 1 bis 3 Kohlenstoffatomen, die gegebenenfalls durch eine Hydroxygruppe, die Alkoxycarbonylgruppe mit 1 bis 2 Kohlenstoffatomen im Alkoholteil substituiert sein kann, eine gegebenenfalls im Phenylrest durch Amino substituierte Benzylgruppe, einen Phenacylrest, einen Oxoalkylrest mit bis zu 4 Kohlenstoffatomen sowie einen Cyclopropylmethylrest steht, ferner einen Rest $COR^4$ oder $SO_2R^5$ bedeutet, wobei

$R^4$ Wasserstoff, gegebenenfalls durch einen Substituenten aus der Reihe Amino oder Carboxy substituiertes Alkyl mit 1 bis 2 C-Atomen, Alkoxy mit 1 bis 2 C-Atomen oder Benzyloxy und

$R^5$ Methyl bedeutet.

Weiterhin wurde gefunden, daß man 7-Amino-1-cyclopropyl-6,8-difluor-1,4-dihydro-4-oxo-3-chinolincarbonsäuren der Formel (I) erhält, wenn man die Trifluor-chinoloncarbonsäure der Formel (II)

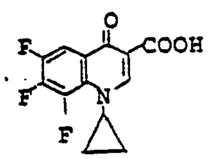
(II)

mit Aminen der Formel (III)

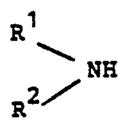
(III)

in welcher $R^1$ und $R^2$ die oben angegebene Bedeutung haben, gegebenenfalls in Gegenwart von Säurebindern umsetzt (Methode A).

Erfindungsgemäße Verbindungen der Formel (I) können auch erhalten werden, indem man eine 7-Piperazinylchinoloncarbonsäure der Formel (IV)

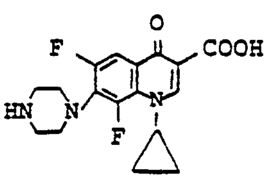
(IV)

in welcher der Piperazinylrest an den Kohlenstoffatomen 1-3 fach durch $C_1$-$C_4$-Alkyl substituiert sein kann, wobei jeweils ein Kohlenstoffatom nur einen Substituenten tragen kann, mit Verbindungen der Formel (V)

$$R^3X \qquad (V)$$

in welcher

$R^3$ die oben angegebene Bedeutung hat, jedoch nicht Wasserstoff sein kann, und

X Fluor, Chlor, Brom, Iod, Acyloxy, Ethoxy, Phenoxy, 4-Nitrophenoxy bedeutet,

in Gegenwart von Säurebindern umsetzt (Methode B).

Man erhält erfindungsgemäße Verbindungen der Formel (I) auch, wenn man eine 7-Piperazinylchinoloncarbonsäure der Formel (IV) in welcher der Piperazinylrest an den Kohlenstoffatomen 1-3 fach durch $C_1$-$C_4$-Alkyl substituiert sein kann, wobei jeweils ein Kohlenstoffatom nur einen Substituenten tragen kann, mit Anhydriden der Formel (VI)

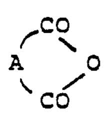
(VI)

in welcher A eine gegebenenfalls substituierte Alkylenkette mit 2 oder 3 Kohlenstoffatomen oder einen Arylenrest bedeutet, zu den erfindungsgemäßen Verbindungen der Formel (Ia) umsetzt (Methode C),

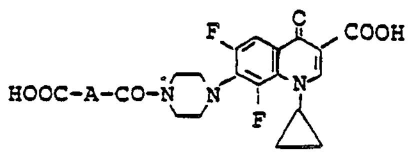

(Ia)

in welcher der Piperazinylrest an den Kohlenstoffatomen 1 bis 3 fach durch $C_1$-$C_4$-Alkyl substituiert sein kann, wobei jeweils ein Kohlenstoffatom nur einen Substituenten tragen kann.

Man erhält erfindungsgemäße Verbindungen der Formel (I) auch, wenn man eine 7-Piperazinyl-chinoloncarbonsäure der Formel (IV) in welcher der Piperazinylrest an den Kohlenstoffatomen 1 bis 3 fach durch $C_1$-$C_4$-Alkyl substituiert sein kann, wobei jeweils ein Kohlenstoffatom nur einen Substituenten tragen kann, mit Michael-Acceptoren der Formel (VII)

$$B{-}CH{=}CH_2 \qquad (VII)$$

in der B für CN, CO—$R^6$ oder $COOR^7$ steht, wobei $R^6$ für Methyl oder Ethyl und $R^7$ für Methyl, Ethyl, n- oder i-Propyl oder Benzyl steht, umsetzt (Methode D).

Verwendet man bei der Umsetzung nach Methode A 2-Methylpiperazin und 1-Cyclopropyl-6,7,8-trifluor-4-oxo-1,4-dihydro-3-chinolincarbonsäure (II) als Ausgangsstoffe, so kann der Reaktionsablauf durch das folgende Formelschema wiedergegeben werden :

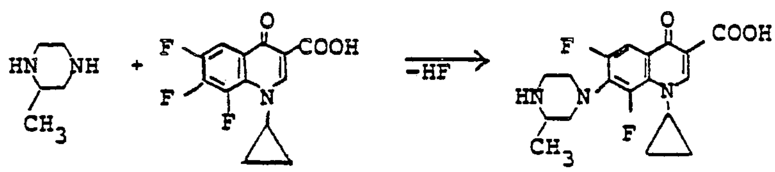


Verwendet man bei der Umsetzung nach Methode B Ethyliodid und 1-Cyclopropyl-6,8-difluor-1,4-dihydro-4-oxo-7-(1-piperazinyl)-3-chinolincarbonsäure als Ausgangsstoffe, so kann der Reaktionsablauf durch das folgende Formelschema wiedergegeben werden :

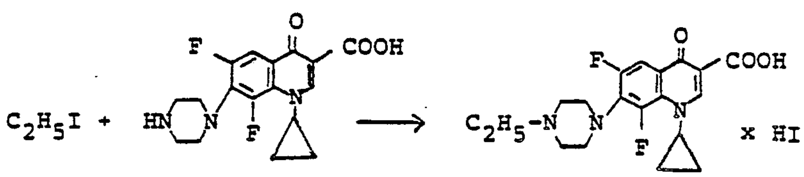

Verwendet man beispielsweise bei der Umsetzung von (IV) mit (V) nach Methode B 1-Cyclopropyl-6,8-difluor-1,4-dihydro-4-oxo-7-(1-piperazinyl)-3-chinolincarbonsäure und Essigsäureanhydrid als Ausgangsverbindungen, so kann der Reaktionsablauf durch das folgende Formelschema wiedergegeben werden :

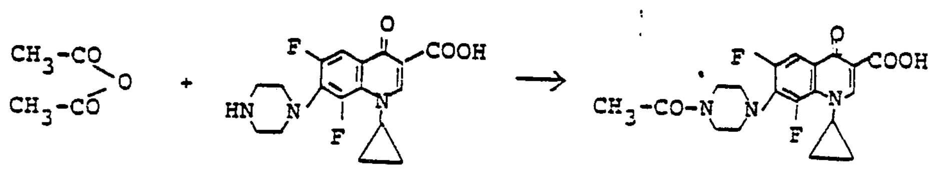

Verwendet man beispielsweise bei der Umsetzung von (I) mit (VI) nach Methode C 1-Cyclopropyl-6,8-difluor-1,4-dihydro-4-oxo-7-(1-piperazinyl)-3-chinolincarbonsäure und Bernsteinsäureanhydrid als Ausgangsverbindungen, so kann der Reaktionsablauf durch das folgende Formelschema wiedergegeben werden :

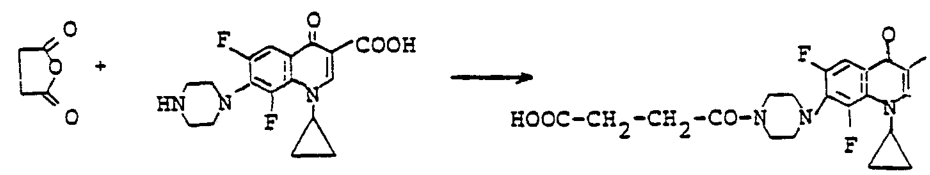

Verwendet man beispielsweise bei der Umsetzung von (I) mit (VII) nach Methode D 1-Cyclopropyl-6,8-difluor-1,4-dihydro-4-oxo-7-(1-piperazinyl)-3-chinolincarbonsäure und Methylvinylketon als Ausgangsverbindungen, so kann der Reaktionsablauf durch das folgende Formelschema wiedergegeben werden :

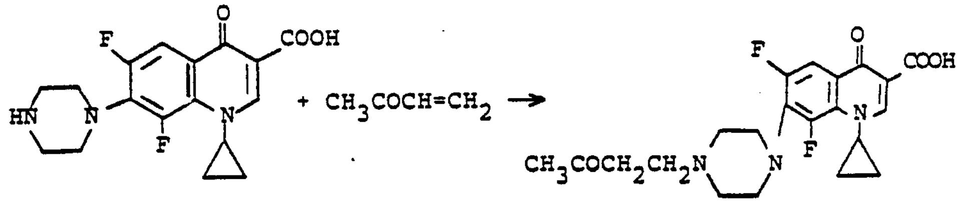

Die als Ausgangsstoffe nach Methode A verwendbare 1-Cyclopropyl-6,7,8-trifluor-1,4-dihydro-4-oxo-3-chinolincarbonsäure der Formel (II) kann gemäß folgendem Reaktionsschema hergestellt werden :

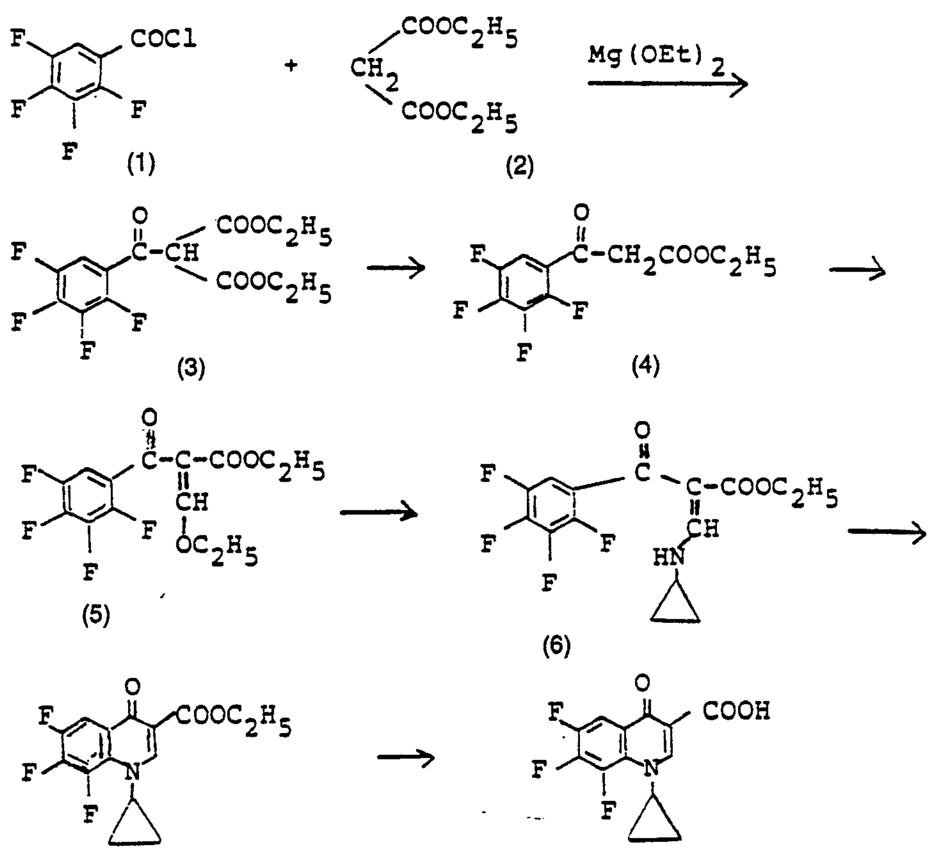


(1) (2) (3) (4) (5) (6) (7) (II)

Danach wird Malonsäurediethylester (2) in Gegenwart von magnesiumethylat mit 2,3,4,5-Tetrafluorbenzoylchlorid (1) zum Aroylmalonester (3) acyliert (Organicum, 3. Aufl. 1964, S. 438).

An Stelle von (1) kann auch das 2,3,4,5-Tetrafluorbenzoesäurefluorid verwendet werden.

Durch partielle Verseifung und Decarboxylierung von (3) in wäßrigem Medium mit katalytischen Mengen Schwefelsäure oder p-Toluolsulfonsäure erhält man in guter Ausbeute den Aroylessigsäureethylester (4), der mit o-Ameisensäure-triethylester/Acetanhydrid in den 2-(2,3,4,5-Tetrafluorbenzoyl)-3-ethoxy-acrylsäureethylester (5) übergeht. Die Umsetzung von (5) mit Cyclopropylamin in einem Lösungsmittel, wie z. B. Methylenchlorid, Alkohol, Chloroform, Cyclohexan oder Toluol führt in leicht exothermer Reaktion zum gewünschten Zwischenprodukt (6).

Die Cyclisierungsreaktion (6) → (7) wird in einem Temperaturbereich von etwa 60 bis 300 °C, bevorzugt 80 bis 180 °C durchgeführt.

Als Verdünnungsmittel können Dioxan, Dimethylsulfoxid, N-Methylpyrrolidon, Sulfolan, Hexamethylphosphorsäuretriamid und bevorzugt N,N-Dimethylformamid verwendet werden.

Als Säurebinder kommen für diese Reaktionsstufe Kalium-tert.-Butanolat, Butyl-lithium, Lithiumphenyl, Phenylmagnesiumbromid, Natriummethylat, Natriumhydrid, Natrium- oder Kalium-carbonat und besonders bevorzugt Kalium- oder Natrium-fluorid in Betracht. Es kann vorteilhaft sein, einen Überschuß von 10 Mol-% an Base einzusetzen.

Die in dem letzten Schritt erfolgende Esterhydrolyse von (7) unter basischen oder sauren Bedingungen führt zu 1-Cyclopropyl-6,7,8-trifluor-1,4-dihydro-4-oxo-3-chinolincarbonsäure (II).

Das als Ausgangsmaterial für diesen Syntheseweg verwendete 2,3,4,5-Tetrafluorbenzoylchlorid (1) wurde aus der literaturbekannten 2,3,4,5-Tetrafluor-benzoesäure [G. G. Yakobson, V. N. Odinokov und N. N. Vorozhtsov Jr, Zh. Obsh. Khim. 36, 139 (1966)] mit Thionylchlorid auf übliche Weise erhalten. Es besitzt einen Siedepunkt von 75-80 °C/17 mbar. Das 2,3,4,5-Tetrafluorbenzoylfluorid besitzt einen Siedepunkt von 46 bis 47 °C/20 mbar ($n_D^{20}$ : 1,437 5).

Die als Ausgangsstoffe verwendeten Amine (III) sind bekannt oder können nach literaturbekannten Verfahren erhalten werden. Als Beispiele seien genannt : Morpholin, Piperidin, Thiomorpholin, Pyrrolidin, Dimethylamin, Ethyl-methylamin, Piperazin, N-Methylpiperazin, N-Ethylpiperazin, N-β-Hydroxyethylpiperazin, N-Formylpiperazin, 2-Methylpiperazin, 1,2-Dimethylpiperazin, cis- und trans-2,5-Dimethyl-piperazin, cis- und trans-2,6-Dimethylpiperazin, 2-Ethylpiperazin, 2-Propylpiperazin, 2-Isopropylpiperazin, 2-Isobutylpiperazin.

Die als Ausgangsstoffe verwendeten Verbindungen der Formel (V) sind bekannt. Als Beispiele seien genannt :

Methyliodid, Methylbromid, Ethyliodid, Ethylbromid, Ethylchlorid, 2-Hydroxyethylchlorid, 3-Hydroxypropylchlorid, 4-Hydroxybutylchlorid, n-Propylbromid, i-Propyliodid, n-Butylbromid, i-Butylbromid, sek.-Butylchlorid, n-Pentylchlorid, 3-Methylbutylchlorid, n-Hexylbromid.

Ameisensäureessigsäureanhydrid, Essigsäureanhydrid, Propionsäureanhydrid, Acetylchlorid, Chloracetylchlorid, Dichloracetylchlorid, Bromacetylbromid, Buttersäurechlorid, 4-Chlorbuttersäurechlorid, Isobuttersäurechlorid, 3-Methylbutansäurechlorid, Benzoylchlorid, 3-Chlorbenzoylchlorid, 4-Fluorbenzoylchlorid, 4-Nitrobenzoylchlorid, 4-Methylbenzoylchlorid, Bernsteinsäuremonomethylester-monochlorid, Trifluormethylthioessigsäurefluorid, N-(tert.-Butoxycarbonyl)-glycin-4-nitrophenylester, N-(tert.-Butoxycarbonyl)-L-alanin-4-nitro-phenylester, N-(tert.-Butoxycarbonyl)-L-leucin-4-nitrophenylester, N-(tert.-Butoxycarbonyl)-L-valin-4-nitrophenylester, 3-Methoxypropionsäurechlorid, Chlorkohlensäuremethylester, Chlorkohlensäureethylester, Chlorkohlensäure-n-butylester, Diethylcarbonat, Chlorcyan, Diphenylcarbonat, Bromcyan, Dimethylcarbamidsäurechlorid, Methansulfonsäurechlorid, Ethansulfonsäurechlorid, Propan-1-sulfonsäurechlorid, Benzolsulfonsäurechlorid, 4-Toluolsulfonsäurechlorid, Butan-1-sulfonsäurechlorid, Dichlorfluormethansulfonsäurechlorid.

Die erfindungsgemäß verwendbaren Anhydride (VI) sind bekannt. Als Beispiele seien genannt :

Bernsteinsäureanhydrid, Methylbernsteinsäureanhydrid, Glutarsäureanhydrid, Phthalsäureanhydrid, Tetrachlorphthalsäureanhydrid.

Die erfindungsgemäß verwendbaren Verbindungen der Formel (VII) sind bekannt. Als Beispiele seien genannt :

Acrylnitril, Methylvinylketon, Acrylsäuremethylester, Acrylsäureethylester, Acrylsäurebenzylester.

Die Umsetzung von (II) mit (III) gemäß Methode A wird vorzugsweise in einem Verdünnungsmittel wie Dimethylsulfoxid, N,N-Dimethylformamid, Hexamethyl-phosphorsäuretrisamid, Sulfolan, Wasser, einem Alkohol wie Methanol, Ethanol, n-Propanol, Isopropanol, Glykolmonomethylether oder Pyridin vorgenommen. Ebenso können Gemische dieser Verdünnungsmittel verwendet werden.

Als Säurebinder können alle üblichen anorganischen und organischen Säurebindungsmittel verwendet werden. Hierzu gehören vorzugsweise die Alkalihydroxide, Alkalicarbonate, organische Amine und Amidine. Als besonders geeignet seien im einzelnen genannt : Triethylamin, 1,4-Diaza-bicyclo [2,2,2]-octan (DABCO), überschüssiges Amin (III) oder 1,8-Diazabicyclo [5,4,0] undec-7-en (DBU).

Die Reaktionstemperaturen können in einem größeren Bereich variiert werden. Im allgemeinen arbeitet man zwischen etwa 20 und 200 °C, vorzugsweise zwischen 80 und 180 °C.

Die Umsetzung kann bei Normaldruck, aber auch bei erhöhtem Druck durchgeführt werden. Im allgemeinen arbeitet man bei Drucken zwischen etwa 1 und etwa 100 bar, vorzugsweise zwischen 1 und 10 bar.

Bei der Durchführung des erfindungsgemäßen Verfahrens setzt man auf 1 Mol der Carbonsäure (II) 1 bis 15 Mol, vorzugsweise 1 bis 6 Mol des Amins (III) ein.

Die Umsetzung von (IV) mit (V) wird vorzugsweise in einem Verdünnungsmittel wie Dimethylsulfoxid, Dioxan, N,N-Dimethylformamid, Hexamethyl-phosphorsäure-tris-amid, Sulfolan, Wasser, einem Alkohol wie Methanol, Ethanol, n-Propanol, Isopropanol, Glykolmonomethylether oder Pyridin vorgenommen. Ebenso können Gemische dieser Verdünnungsmittel verwendet werden.

Als Säurebinder können alle üblichen anorganischen und organischen Säurebindungsmittel verwendet werden. Hierzu gehören vorzugsweise die Alkalihydroxide, Alkalicarbonate, organische Amine und Amidine. Als besonders geeignet seien im einzelnen genannt : Triethylamin, 1,4-Diazabicyclo [2,2,2] octan (DABCO) oder 1,8-Diazabicyclo-[5,4,0] undec-7-en (DBU).

Die Reaktionstemperaturen können in einem größeren Bereich variiert werden. Im allgemeinen arbeitet man zwischen etwa 20 und etwa 180 °C, vorzugsweise zwischen 40 und 110 °C.

Die Umsetzung kann bei Normaldruck, aber auch bei erhöhtem Druck durchgeführt werden. Im allgemeinen arbeitet man bei Drucken zwischen etwa 1 und etwa 100 bar, vorzugsweise zwischen 1 und 10 bar.

Bei der Durchführung des erfindungsgemäßen Verfahrens gemäß Methode B setzt man auf 1 Mol der Verbindung (IV) 1 bis 4 Mol, vorzugsweise 1 bis 1,5 Mol der Verbindung (V) ein.

Die Umsetzung von (IV) mit (VI) (Methode C) wird in einem Verdünnungsmittel wie N,N-Dimethylformamid, Dioxan, Tetrahydrofuran, Pyridin, Wasser oder auch in Mischungen dieser Verdünnungsmittel durchgeführt. Die Reaktionstemperaturen können in einem größeren Bereich variiert werden. Im allgemeinen arbeitet man zwischen etwa 0 °C und etwa 140 °C, vorzugsweise zwischen 10 °C und 100 °C.

Die Umsetzung kann bei Normaldruck, aber auch bei erhöhtem Druck durchgeführt werden. Im allgemeinen arbeitet man bei Drucken zwischen etwa 1 und etwa 100 bar, vorzugsweise zwischen 1 und 10 bar.

Als Säurebinder können alle üblichen anorganischen und organischen Säurebindungsmittel verwendet werden. Hierzu gehören vorzugsweise die Alkalihydroxide, Alkalicarbonate, Pyridin und tert.-Amine wie Triethylamin, 1,4-Diazabicyclo [2,2,2] octan.

Bei der Durchführung des erfindungsgemäßen Verfahrens setzt man auf 1 Mol der Verbindung (IV) 1 bis 3 Mol, vorzugsweise 1 bis 1,3 Mol der Verbindung (VI) ein.

Die Umsetzung von (IV) mit (VII) (Methode D) wird vorzugsweise in einem Verdünnungsmittel wie Dioxan, Dimethylsulfoxid, N,N-Dimethylformamid, Methanol, Ethanol, Isopropanol, n-Propanol, Glykolmonomethylether oder auch in Gemischen dieser Verdünnungsmittel durchgeführt.

Die Reaktionstemperaturen können in einem größeren Bereich variiert werden. Im allgemeinen arbeitet man zwischen etwa 20 °C und etwa 150 °C, vorzugsweise zwischen 50 °C und 100 °C.

Die Umsetzung kann bei Normaldruck, aber auch bei erhöhtem Druck durchgeführt werden. Im allgemeinen arbeitet man bei Drucken zwischen etwa 1 und etwa 100 bar, vorzugsweise zwischen 1 und 10 bar.

Bei der Durchführung des erfindungsgemäßen Verfahrens setzt man auf 1 Mol der Verbindung (IV) 1 bis 5 Mol, vorzugsweise 1 bis 2 Mol, der Verbindung (VII) ein.

Als neue Wirkstoffe seien im einzelnen genannt :

1-Cyclopropyl-6,8-difluor-1,4-dihydro-4-oxo-7-(1-piperazinyl)-3-chinolincarbonsäure,
1-Cyclopropyl-6,8-difluor-1,4-dihydro-4-oxo-7-(4-methyl-1-piperazinyl)-3-chinolincarbonsäure,
1-Cyclopropyl-6,8-difluor-1,4-dihydro-4-oxo-7-(4-ethyl-1-piperazinyl)-3-chinolincarbonsäure,
1-Cyclopropyl-6,8-difluor-1,4-dihydro-4-oxo-7-(3-methyl-1-piperazinyl)-3-chinolincarbonsäure,
1-Cyclopropyl-6,8-difluor-1,4-dihydro-4-oxo-7-(3,4-dimethyl-1-piperazinyl)-3-chinolincarbonsäure,
1-Cyclopropyl-6,8-difluor-1,4-dihydro-4-oxo-7-(4-ethyl-3-methyl-1-piperazinyl)-3-chinolincarbonsäure,
1-Cyclopropyl-6,8-difluor-1,4-dihydro-4-oxo-7-[4-(2-hydroxyethyl)-3-methyl-1-piperazinyl]-3-chinolincarbonsäure,
1-Cyclopropyl-6,8-difluor-1,4-dihydro-4-oxo-7-[4-(3-hydroxypropyl)-3-methyl-1-piperazinyl]-3-chinolincarbonsäure,
1-Cyclopropyl-6,8-difluor-1,4-dihydro-4-oxo-7-(2,5-dimethyl-1-piperazinyl)-3-chinolincarbonsäure,
1-Cyclopropyl-6,8-difluor-1,4-dihydro-4-oxo-7-(4-ethyl-2,5-dimethyl-1-piperazinyl)-3-chinolincarbonsäure,
1-Cyclopropyl-6,8-difluor-1,4-dihydro-4-oxo-7-(3,5-dimethyl-1-piperazinyl)-3-chinolincarbonsäure,
1-Cyclopropyl-6,8-difluor-1,4-dihydro-4-oxo-7-(3,4,5-trimethyl-1-piperazinyl)-3-chinolincarbonsäure,
1-Cyclopropyl-6,8-difluor-1,4-dihydro-4-oxo-7-(4-ethyl-3,5-dimethyl-1-piperazinyl)-3-chinolincarbonsäure,
1-Cyclopropyl-6,8-difluor-1,4-dihydro-4-oxo-7-(3-ethyl-1-piperazinyl)-3-chinolincarbonsäure,
1-Cyclopropyl-6,8-difluor-1,4-dihydro-4-oxo-7-(3-n-propyl-1-piperazinyl)-3-chinolincarbonsäure,
1-Cyclopropyl-6,8-difluor-1,4-dihydro-4-oxo-7-(3-isopropyl-1-piperazinyl)-3-chinolincarbonsäure,
1-Cyclopropyl-6,8-difluor-1,4-dihydro-4-oxo-7-(3-isobutyl-1-piperazinyl)-3-chinolincarbonsäure,
1-Cyclopropyl-6,8-difluor-1,4-dihydro-4-oxo-(3-methyl-4-n-propyl-1-piperazinyl)-3-chinolincarbonsäure,
1-Cyclopropyl-6,8-difluor-1,4-dihydro-4-oxo-7-(3-methyl-4-isopropyl-1-piperazinyl)-3-chinolincarbonsäure,
1-Cyclopropyl-6,8-difluor-1,4-dihydro-4-oxo-7-(4-n-butyl-3-methyl-1-piperazinyl)-3-chinolincarbonsäure,
1-Cyclopropyl-6,8-difluor-1,4-dihydro-4-oxo-7-morpholinyl-3-chinolincarbonsäure,

und deren pharmazeutisch verwendbaren Säureadditionssalze, Alkalisalze, Erdalkalisalze oder Hydrate.

## Herstellungsbeispiele

### Beispiel A (Herstellung des Ausgangsproduktes II) :

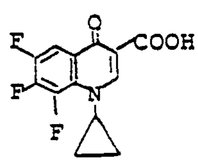

24,3 g Magnesiumspäne werden in 50 ml wasserfreiem Ethanol suspendiert. Man versetzt mit 5 ml Tetrachlorkohlenstoff und tropft, wenn die Reaktion in Gang gekommen ist, ein Gemisch von 160 g Malonsäurediethylester, 100 ml abs. Ethanol und 400 ml wasserfreiem Toluol bei 50-60 °C zu. Dann wird noch 1 Stunde auf 50-60 °C erhitzt, mit Trockeneis/Aceton auf — 5 °C bis — 10 °C gekühlt und bei dieser Temperatur eine Lösung von 212,5 g 2,3,4,5-Tetrafluorbenzoylchlorid (1) in 80 ml abs. Toluol langsam zugetropft. Man rührt 1 Stunde bei 0 bis — 5 °C, läßt über Nacht auf Raumtemperatur kommen und läßt unter Eiskühlung ein Gemisch von 400 ml Eiswasser und 25 ml konz. Schwefelsäure zulaufen. Die Phasen werden getrennt und zweimal mit Toluol nachextrahiert. Die vereinigten Toluollösungen werden mit gesättigter NaCl-Lösung gewaschen, mit $Na_2SO_4$ getrocknet und das Lösungsmittel im Vakuum abgezogen. Man erhält 335 g 2,3,4,5-Tetra-fluorbenzoyl-malonsäurediethylester (3) als Rohprodukt.

Eine Emulsion von 284,8 g rohem 2,3,4,5-Tetrafluorbenzoyl-malonsäurediethylester (3) in 300 ml Wasser wird mit 0,3 g p-Toluolsulfonsäure versetzt. Man erhitzt unter gutem Rühren 5 Stunden zum Sieden, extrahiert die erkaltete Emulsion mehrmals mit Methylenchlorid, wäscht die vereinigten $CH_2Cl_2$-Lösungen einmal mit gesättigter NaCl-Lösung, trocknet mit $Na_2SO_4$ und destilliert das Lösungsmittel im Vakuum ab. Die Fraktionierung des Rückstandes im Hochvakuum liefert 160,2 g 2,3,4,5-Tetrafluorbenzoyl-essigsäureethylester (4) vom Siedepunkt 100-110 °C/0,09-0,1 mbar. Schmelzpunkt 47-49 °C.

Ein Gemisch von 110,7 g 2,3,4,5-Tetrafluorbenzoylessigsäureethylester (4), 93,5 g o-Ameisensäureethylester und 107 g Acetanhydrid wird 2 Stunden auf 150 °C erhitzt. Dann werden im Wasserstrahlvakuum und zuletzt im Hochvakuum bei einer Badtemperatur von ~ 120 °C die flüchtigen Bestandteile abdestilliert. Zurück bleiben 123,9 g roher 2-(2,3,4,5-Tetrafluorbenzoyl)-3-ethoxyacrylsäureethylester (5). Er ist genügend rein für die weiteren Umsetzungen.

Eine Lösung von 123,9 g 2-(2,3,4,5-Tetrafluorbenzoyl)-3-ethoxyacrylsäureethylester (5) in 250 ml Ethanol wird unter Eiskühlung und Rühren tropfenweise mit 23,2 g Cyclopropylamin versetzt. Wenn die exotherme Reaktion abgeklungen ist, wird noch 1 Stunde bei Raumtemperatur gerührt, das Lösungsmittel im Vakuum abgezogen und der Rückstand aus Cyclohexan/Petrolether umkristallisiert. Man erhält 115 g 2-(2,3,4,5-Tetrafluorbenzoyl)-3-cyclopropylamino-acrylsäureethylester (6) vom Schmelzpunkt 63-65 °C.

Eine Lösung von 107,8 g 2-(2,3,4,5-Tetrafluorbenzoyl)-3-cyclopropylamino-acrylsäureethylester (6) in 400 ml wasserfreiem Dimethylformamid wird mit 21,2 g Natriumfluorid versetzt. Dann wird 2 Stunden unter Rückfluß gerührt und das Reaktionsgemisch heiß auf Eis gegossen. Der Niederschlag wird abgesaugt, mit Wasser gut gewaschen und im Vakuum über Calciumchlorid bei 100 °C getrocknet. Man erhält 91,2 g 1-Cyclopropyl-6,7,8-trifluor-1,4-dihydro-4-oxo-3-chinolincarbonsäureethylester (7) vom Schmelzpunkt 167-168 °C.

Ein Gemisch von 94 g 1-Cyclopropyl-6,7,8-trifluor-1,4-dihydro-4-oxo-3-chinolincarbonsäureethylester (7), 600 ml Eisessig, 450 ml Wasser und 70 ml konz. Schwefelsäure wird 1,5 Stunden auf Rückfluß erhitzt. Dann gießt man die heiße Suspension auf Eis, saugt den Niederschlag ab, wäscht gut mit Wasser nach und trocknet im Vakuum bei 100 °C. Man erhält auf diese Weise 88,9 g reine 1-Cyclopropyl-6,7,8-trifluor-1,4-dihydro-4-oxo-3-chinolincarbonsäure II vom Schmelzpunkt 228-230 °C (Zersetzung).

## Beispiel 1

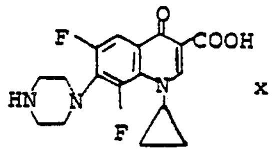

x HCl

Ein Gemisch aus 2,83 g (0,01 Mol) 1-Cyclopropyl-6,7,8-trifluor-1,4-dihydro-4-oxo-3-chinolincarbonsäure (II), 4,4 g (0,051 Mol) wasserfreies Piperazin und 30 ml trockenem Pyridin wird 6 Stunden refluxiert. Das Lösungsmittel wird im Vakuum abgezogen, der Rückstand in 25 ml Wasser aufgenommen, unter Eiskühlung mit konzentrierter Salzsäure auf pH 1 gestellt, der Niederschlag kalt abgesaugt, mit kalter 10 %iger Salzsäure und Ethanol gewaschen. Nach dem Trocknen im Vakuum bei 100 °C erhält man 3,05 g 1-Cyclopropyl-6,8-difluor-7-(1-piperazinyl)-1,4-dihydro-4-oxo-3-chinolincarbonsäure-hydrochlorid vom Zersetzungspunkt 354-355 °C.

## Beispiel 2

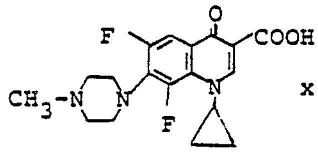

x HCl

Man setzt 2,83 g (0,01 Mol) II mit 4 g (0,04 Mol) N-Methylpiperazin analog Beispiel 1 um und isoliert 3,6 g 1-Cyclopropyl-6,8-difluor-7-(4-methyl-1-piperazinyl)-1,4-dihydro-4-oxo-3-chinolincarbonsäure-hydrochlorid vom Zersetzungspunkt 300-303 °C.

Analog Beispiel 1 bzw. 2 werden folgende Verbindungen erhalten :

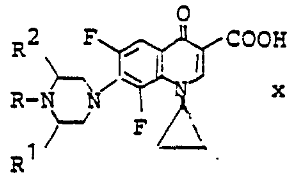

x HCl

| Beispiel | R | $R^1$ | $R^2$ | Zersetzungspunkt (°C) |
|---|---|---|---|---|
| 3 | H | $CH_3$ | H | 325-330 |
| 4 | H | $C_2H_5$ | H | 330-335 |
| 5 | H | $CH_3$ | $CH_3$ | 310-315 |
| 6 | $HO(CH_2)_2$- | H | H | 290-293 |

Beispiel 7

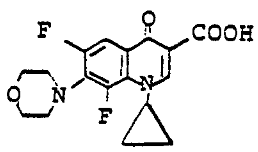

Ein Gemisch von 2,83 g (0,01 Mol) 1-Cyclopropyl-6,7,8-trifluor-1,4-dihydro-4-oxo-3-chinolincarbonsäure, 0,9 g (0,01 Mol) Morpholin und 2,3 g Diaza-bicyclo [2,2,2]-octan (0,02 Mol) in 35 ml Dimethylsulfoxid wird 5 Stunden auf 140 °C erhitzt. Das Lösungsmittel wird im Vakuum abdestilliert, der Rückstand mit 50 ml Wasser versetzt, mit halbkonzentrierter Salzsäure angesäuert, kalt abgesaugt, mit Wasser gewaschen, im Vakuum getrocknet und aus Glykolmonomethylether umkristallisiert. Man erhält 2,4 g 1-Cyclopropyl-6,8-difluor-7-(4-morpholinyl)-1,4-dihydro-4-oxo-3-chinolincarbonsäure vom Zersetzungspunkt 257-260 °C.

Analog Beispiel 7 werden folgende Verbindungen erhalten :

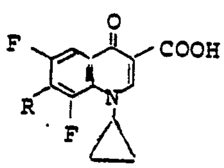

| Beispiel | R | Zersetzungspunkt (°C) |
|---|---|---|
| 8 | 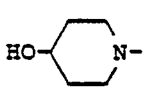 | 277-280 |
| 9 | 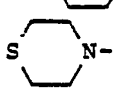 | 291-294 |
| 10 | 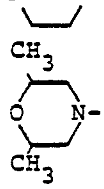 | 241-245 |
| 11 | 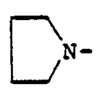 | 280-285 |

Beispiel 12

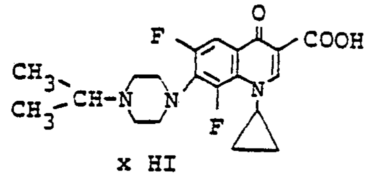

x HI

Man erhitzt eine Mischung von 3,5 g (0,01 Mol) 1-Cyclopropyl-6,8-difluor-7-(1-piperazinyl)-1,4-dihydro-4-oxo-3-chinolincarbonsäure, 3,4 g (0,02 Mol) Isopropyliodid und 2,1 g (0,02 Mol) Triethylamin in 50 ml Dimethylformamid während 6 Stunden auf 80 °C. Nach dem Abdampfen des Lösungsmittels wird mit 30 ml Wasser verrührt, der Rückstand abgesaugt, mit Wasser gewaschen und aus Methanol umkristallisiert. Man erhält 2,3 g 1-Cyclopropyl-6,8-difluor-7-(4-isopropyl-1-piperazinyl)-1,4-dihydro-4-oxo-3-chinolincarbonsäure-hydroiodid mit einem Zersetzungspunkt von 306-308 °C.

Analog Beispiel 12 werden die folgenden Verbindungen erhalten :

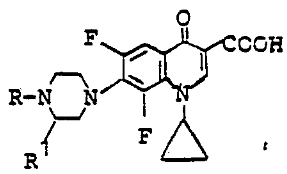

x HI

| Beispiel | R | $R^1$ | Zersetzungspunkt (°C) |
|---|---|---|---|
| 13 | $C_2H_5$ | H | 289-291 |
| 14 | $C_2H_5$ | $CH_3$ | 252-258 |

Beispiel 15

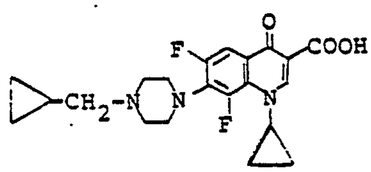

Eine Mischung aus 3,5 g (0,01 Mol) 1-Cyclopropyl-6,8-difluor-7-(1-piperazinyl)-1,4-dihydro-4-oxo-3-chinolincarbonsäure, 2,1 g Triethylamin, 1,8 g Cyclopropylmethyl-chlorid und 3,3 g Kaliumiodid wird 6 Stunden auf 80 °C erhitzt. Anschließend wird im Vakuum eingeengt, mit 30 ml Wasser versetzt, die Mischung auf pH 5 eingestellt, der Niederschlag abgesaugt, mit Wasser und Methanol gewaschen und aus Glykolmonomethylether umkristallisiert. Man erhält 1,8 g 1-Cyclopropyl-7-(4-cyclopropylmethyl-1-piperazinyl)-6,8-difluor-1,4-dihydro-4-oxo-3-chinolincarbonsäure mit einem Zersetzungspunkt von 246-248 °C.

Analog Beispiel 15 werden die folgenden Verbindungen erhalten :

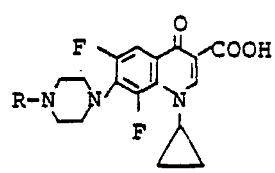

| Beispiel | R | Zersetzungspunkt (°C) |
|---|---|---|
| 16 | 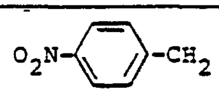 | 210-212 |
| 17 | $CH_3-CO-CH_2$ | 201-204 |
| 18 | 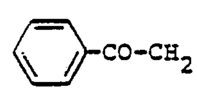 | 210-212 |

(Fortsetzung)

| Beispiel | R | Zersetzungspunkt (°C) |
|---|---|---|
| 19 | $CH_2=CH-CH_2$ | 172-175 |
| 20 | $HC\equiv C-CH_2$ | 228-232 (Hydrochlorid) |

Beispiel 21

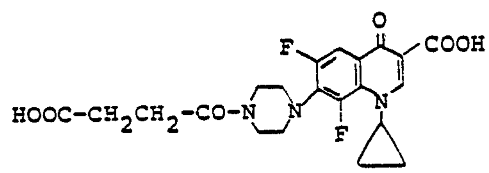

Man löst 3,5 g (0,01 Mol) 1-Cyclopropyl-6,8-difluor-7-(1-piperazinyl)-1,4-dihydro-4-oxo-3-chinolincarbonsäure in einer Mischung von 0,4 g Natriumhydroxid und 20 ml Wasser und tropft bei 20 °C gleichzeitig eine Lösung von 1 g Bernsteinsäureanhydrid in 10 ml Dioxan und eine Lösung von 0,4 g Natriumhydroxid in 10 ml Wasser zu. Nach 12-stündigem Stehen wird mit 2n-Salzsäure auf pH 5 eingestellt, der Niederschlag abgesaugt, mit Wasser gewaschen, mit Methanol erhitzt, abgesaugt und getrocknet. Man erhält 2,5 g 7-[4-(3-Carboxypropionyl)-1-piperazinyl]-1-cyclopropyl-6,8-difluor-1,4-dihydro-4-oxo-3-chinolincarbonsäure mit einem Zersetzungspunkt von 217-219 °C.

Analog Beispiel 21 werden erhalten :

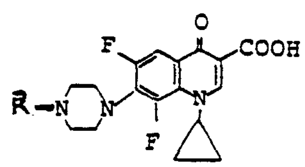

| Beispiel | R | Acylierungsmittel | Zersetzungspunkt (°C) |
|---|---|---|---|
| 22 | H-CO | $HCO-O-COCH_3$ | 295-300 |
| 23 | $CH_3$-CO | $CH_3-CO-Cl$ | 248-251 |
| 24 | n-$C_3H_7$-CO | $C_3H_7-CO-Cl$ | 182-186 |
| 25 | $C_2H_5O$-CO | $C_2H_5O-CO-Cl$ | 204-208 |
| 26 | $CH_3-SO_2$ | $CH_3-SO_2-Cl$ | 295-296 |

Beispiel 27

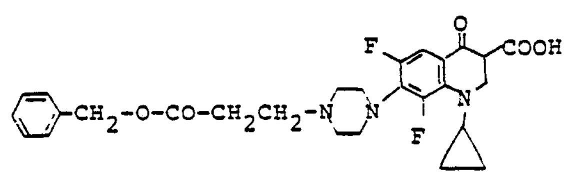

3,5 g (0,01 Mol) 1-Cyclopropyl-6,8-difluor-7-(1-piperazinyl)-1,4-dihydro-4-oxo-3-chinolincarbonsäure werden mit 4 g Acrylsäurebenzylester 5 Stunden unter Rückfluß erhitzt. Die Lösung wird heiß filtriert, der ausgefallene Niederschlag abgesaugt, mit Ethanol gewaschen und getrocknet. Man erhält 2,3 g 7-[4-(2-Benzyloxycarbonyl-ethyl)-1-piperazinyl]-1-cyclopropyl-6,8-difluor-1,4-dihydro-4-oxo-3-chinolincarbonsäure mit einem Zersetzungspunkt von 132-135 °C.

Beispiel 28

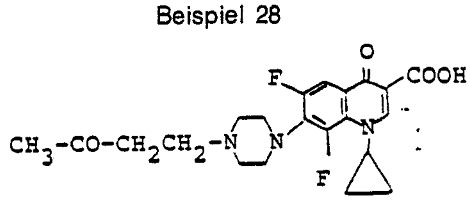

Man arbeitet analog Beispiel 27 unter Verwendung von Methyl-vinylketon und erhält 1-Cyclopropyl-6,8-difluor-1,4-dihydro-4-oxo-7-[4-(3-oxobutyl)-1-piperazinyl]-3-chinolincarbonsäure mit einem Zersetzungspunkt von 155-158 °C.

Die erfindungsgemäßen Verbindungen zeigen bei geringer Toxizität ein breites antibakterielles Spektrum gegen gram-positive und gram-negative Keime, insbesondere gegen Enterobakteriaceen ; vor allem auch gegen solche, die resistent sind gegen verschiedene Antibiotika, wie z. B. Penicilline, Cephalosporine, Aminoglykoside, Sulfonamide, Tetracycline.

Diese wertvollen Eigenschaften ermöglichen ihre Verwendung als chemotherapeutische Wirkstoffe in der Medizin sowie als Stoffe zur Konservierung von anorganischen und organischen Materialien, insbesondere von organischen Materialien aller Art, z. B. Polymeren, Schmiermitteln, Farben, Fasern, Leder, Papier und Holz, von Lebensmitteln und von Wasser.

Die erfindungsgemäßen Verbindungen sind gegen ein sehr breites Spektrum von Mikroorganismen wirksam. Mit ihrer Hilfe können gram-negative und gram-positive Bakterien und bakterienähnliche Mikroorganismen bekämpft sowie die durch diese Erreger hervorgerufenen Erkrankungen verhindert, gebessert und/oder geheilt werden.

Besonders wirksam sind die erfindungsgemäßen Verbindungen gegen Bakterien und bakterienähnliche Mikroorganismen. Sie sind daher besonders gut zur Prophylaxe und Chemotherapie von lokalen und systemischen Infektionen in der Human- und Tiermedizin geeignet, die durch diese Erreger hervorgerufen werden.

Beispielsweise können lokale und/oder systemische Erkrankungen behandelt und/oder verhindert werden, die durch die folgenden Erreger oder durch Mischungen der folgenden Erreger verursacht werden :

Micrococcaceae, wie Staphylokokken, z. B. Staphylococcus aureus, Staph. Epidermidis, (Staph. = Staphylococcus) ; Lactobacteriaceae, wie Streptokokken, z. B. Streptococcus pyogenes, α- bzw. β-hämolysierende Streptokokken, nicht (γ-) hämolysierende Streptokokken, Enterokokken und Diplococcus pneumoniae (Pneumokokken) ;

Enterobacteriaceae, wie Escherichiae-Bakterien der Coli-Gruppe : Escherichia-Bakterien, z. B. Escherichia coli, Enterobacter-Bakterien, z. B. E. aerogenes, E. Cloacae, Klebsiella-Bakterien, z. B. K. pneumoniae, Serratia, z. B. Serratia marcescens (E. = Enterobacter) (K. = Klebsiella), Proteae-Bakterien der Proteus-Gruppe : Proteus, z. B. Proteus vulgaris, Pr. morganii, Pr. rettgeri, Pr. mirabilis (Pr. = Proteus) ;

Pseudomonadaceae, wie Pseudomonas-Bakterien, z. B. Pseudomonas aeruginosa.

Bacteroidaceae, wie Bacteroides-Bakterien, z. B. Bacteroides fragilis (B. = Bacteroides) ; Mykoplasmen, z. B. Mykoplasma pneumoniae.

Die obige Aufzählung von Erregern ist lediglich beispielhaft und keineswegs beschränkend aufzufassen.

Als Krankheiten, die durch die erfindungsgemäßen Verbindungen verhindert, gebessert und/oder geheilt werden können, seien beispielsweise genannt :

Erkrankungen der Atmungswege und des Rachenraumes :

Otitis ; Pharyngitis ; Pneumonie ; Peritonitis ; Pyelonephritis ; Cystitis ; Endocarditis ; Systeminfektionen ; Bronchitis ; Arthritis ; lokale Infektionen, septische Erkrankungen.

Zur Erfindung gehören pharmazeutische Zubereitungen, die neben nicht-toxischen, inerten pharmazeutisch geeigneten Trägerstoffen einen oder mehrere erfindungsgemäße Verbindungen enthalten oder die aus einem oder mehreren erfindungsgemäßen Wirkstoffen bestehen, sowie Verfahren zur Herstellung dieser Zubereitungen.

Zur Erfindung gehören auch pharmazeutische Zubereitungen in Dosierungseinheiten. Dies bedeutet, daß die Zubereitungen in Form einzelner Teile, z. B. Tabletten, Dragees, Kapseln, Pillen, Suppositorien und Ampullen vorliegen, deren Wirkstoffgehalt einem Bruchteil oder einem Vielfachen einer Einzeldosis entspricht. Die Dosierungseinheiten können z. B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nicht-toxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder und Sprays genannt.

Tabletten, Dragees, Kapseln, Pillen und Granulate können den oder die Wirkstoffe neben den üblichen Trägerstoffen enthalten, wie (a) Füll- und Streckmittel, z. B. Stärken, Milchzucker, Rohrzucker, Glukose, Mannit und Kieselsäure, (b) Bindemittel, z. B. Carboxymethylcellulose, Alginate, Gelatine, Polyvinylpyrrolidon, (c) Feuchthaltemittel, z. B. Glycerin, (d) Sprengmittel, z. B. Agar-Agar, Calciumcarbonat und Natriumcarbonat, (e) Lösungsverzögerer, z. B. Paraffin und (f) Resorptionsbeschleuniger, z. B. quaternäre Ammoniumverbindungen, (g) Netzmittel, z. B. Cetylalkohol, Glycerinmonostearat, (h) Adsorptionsmittel, z. B. Kaolin und Bentonit und (i) Gleitmittel, z. B. Talkum, Calcium- und Magnesiumstearat und feste Polyethylenglykole oder Gemische der unter (a) bis (i) aufgeführten Stoffe.

Die Tabletten, Dragees, Kapseln, Pillen und Granulate können mit den üblichen, gegebenenfalls Opakisierungsmittel enthaltenden, Überzügen und Hüllen versehen sein und auch so zusammengesetzt sein, daß sie den oder die Wirkstoffe nur oder bevorzugt in einem bestimmten Teil des Intestinaltraktes gegebenenfalls verzögert abgeben, wobei als Einbettungsmassen z. B. Polymersubstanzen und Wachse verwendet werden können.

Der oder die Wirkstoffe können gegebenenfalls mit einem oder mehreren der oben angegebenen Trägerstoffe auch in mikroverkapselter Form vorliegen.

Suppositorien können neben dem oder den Wirkstoffen die üblichen wasserlöslichen oder wasserunlöslichen Trägerstoffe enthalten, z. B. Polyethylenglykole, Fette, z. B. Kakaofett und höhere Ester (z. B. $C_{14}$-Alkohol mit $C_{16}$-Fettsäure) oder Gemische dieser Stoffe.

Salben, Pasten, Cremes und Gele können neben dem oder den Wirkstoffen die üblichen Trägerstoffe enthalten, z. B. tierische und pflanzliche Fette, Wachse, Paraffine, Stärke, Tragant, Cellulosederivate, Polyethylenglykole, Silikone, Bentonite, Kieselsäure, Talkum und Zinkoxid oder Gemische dieser Stoffe.

Puder und Sprays können neben dem oder den Wirkstoffen die üblichen Trägerstoffe enthalten, z. B. Milchzucker, Talkum, Kieselsäure, Aluminiumhydroxid, Calciumsilikat und Polyamidpulver oder Gemische dieser Stoffe. Sprays können zusätzlich die üblichen Treibmittel, z. B. Chlorfluorkohlenwasserstoffe, enthalten.

Lösungen und Emulsionen können neben dem oder den Wirkstoffen die üblichen Trägerstoffe wie Lösungsmittel, Lösungsvermittler und Emulgatoren, z. B. Wasser, Ethylalkohol, Isopropylalkohol, Ethylcarbonat, Ethylacetat, Benzylalkohol, Benzylbenzoat, Propylenglykol, 1,3-Butylenglykol, Dimethylformamid, Öle, insbesondere Baumwollsaatöl, Erdnußöl, Maiskeimöl, Olivenöl, Ricinusöl und Sesamöl, Glycerin, Glycerinformal, Tetrahydrofurfurylalkohol, Polyethylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe wie flüssige Verdünnungsmittel, z. B. Wasser, Ethylalkohol, Propylenglykol, Suspendiermittel, z. B. ethoxylierte Isostearylalkohole, Polyoxyethylensorbit- und Sorbitan-Ester, mikrokristalline Cellulose, Aluminiummetahydroxid, Bentonit, Agar-Agar und Tragant oder Gemische dieser Stoffe enthalten.

Die genannten Formulierungsformen können auch Färbemittel, Konservierungsstoffe sowie geruchs- und geschmacksverbesserte Zusätze, z. B. Pfefferminzöl und Eukalyptusöl und Süßmittel, z. B. Saccharin, enthalten.

Die therapeutische wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Gew.-%, der Gesamtmischung vorhanden sein.

Die oben aufgeführten pharmazeutischen Zubereitungen können außer den erfindungsgemäßen Verbindungen auch weitere pharmazeutische Wirkstoffe enthalten.

Die Herstellung der oben aufgeführten pharmazeutischen Zubereitungen erfolgt in üblicher Weise nach bekannten Methoden, z. B. durch Mischen des oder der Wirkstoffe mit dem oder den Trägerstoffen.

Die Wirkstoffe oder die pharmazeutischen Zubereitungen können lokal, oral, parenteral, intraperitoneal und/oder rectal, vorzugsweise oral oder parenteral wie intravenös oder intramuskulär appliziert werden.

Im allgemeinen hat es sich sowohl in der Human- als auch in der Veterinärmedizin als vorteilhaft erwiesen, den oder die erfindungsgemäßen Wirkstoffe in Gesamtmengen von etwa 0,5 bis etwa 500, vorzugsweise 5 bis 100 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die erfindungsgemäßen Wirkstoffe, vorzugsweise in Mengen von etwa 1 bis etwa 250, insbesondere 3 bis 60 mg/kg Körpergewicht. Es kann jedoch erforderlich sein, von den genannten Dosierungen abzuweichen, und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt.

So kann es in einigen Fällen ausreichend sein, mit weniger als der obengenannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

Die neuen Verbindungen können in den üblichen Konzentrationen und Zubereitungen zusammen mit dem Futter bzw. mit Futterzubereitungen oder mit dem Trinkwasser gegeben werden. Dadurch kann eine Infektion durch gram-negative oder gram-positive Bakterien verhindert, gebessert und/oder geheilt werden und dadurch eine Förderung des Wachstums und eine Verbesserung der Verwertung des Futters erreicht werden.

In der nachstehenden Tabelle sind die MHK-Werte einiger der erfindungsgemäßen Verbindungen angegeben.

(Siehe Tabelle Seite 14 ff.)

Tabelle

| | MIK mg/ml | | | | | | | | | |
|---|---|---|---|---|---|---|---|---|---|---|
| Stamm | Bsp.13 | Bsp.3 | Bsp.5 | Bsp.4 | Bsp.12 | Bsp.8 | Bsp.28 | Bsp.1 | Bsp.2 | Bsp.11 |
| E.coli Neumann | 0,015 | 0,015 | 0,015 | 0,015 | 0,06 | 0,015 | 0,015 | 0,015 | 0,015 | 0,06 |
| E.coli 455/7 | 4 | 8 | 8 | 8 | 16 | 8 | 8 | 4 | 1 | 128 |
| Klebsiella 63 | 0,03 | 0,015 | 0,015 | 0,06 | 0,125 | 0,125 | 0,015 | 0,015 | 0,015 | 0,5 |
| Klebsiella 8085 | 0,015 | 0,015 | 0,015 | 0,015 | 0,03 | 0,015 | 0,015 | 0,015 | 0,015 | 0,125 |
| Proteus mir. 8223 | 4 | 4 | 4 | 8 | 16 | 4 | 4 | 2 | 2 | 8 |
| Proteus vulg. 1017 | 0,06 | 0,03 | 0,06 | 0,06 | 0,5 | 0,06 | 0,015 | 0,015 | 0,03 | 1 |
| Proteus morg. 932 | 0,015 | 0,015 | 0,015 | 0,015 | 0,06 | 0,03 | 0,015 | 0,015 | 0,015 | 0,5 |
| Staph.aur. 133 | 0,25 | 0,125 | 0,125 | 0,125 | 0,25 | 0,015 | 0,25 | 0,125 | 0,125 | 0,03 |
| Streptococc. faec. 9790 | 1 | 0,5 | 0,5 | 0,5 | 2 | 0,125 | 0,5 | 0,25 | 0,5 | 0,5 |
| Pseudom. W. | 0,5 | 0,5 | 1 | 1 | 8 | 1 | 0,25 | 0,06 | 0,125 | 4 |

Tabelle (Fortsetzung)

| | MIK mg/ml | | | | |
|---|---|---|---|---|---|
| Stamm | Beispiel 15 | Beispiel 14 | Beispiel 6 | Beispiel 7 | Beispiel 10 |
| E.coli Neumann | 0,03 | 0,015 | 0,015 | 0,015 | 0,125 |
| E.coli 455/7 | 16 | 16 | 8 | 16 | 32 |
| Klebsiella 63 | 0,125 | 0,03 | 0,03 | 0,06 | 0,5 |
| Klebsiella 8085 | 0,03 | 0,015 | 0,015 | 0,03 | 0,25 |
| Proteus mir. 8223 | 8 | 16 | 4 | 8 | 8 |
| Proteus vulg. 1017 | 0,5 | 0,25 | 0,06 | 0,25 | 1 |
| Proteus morg. 932 | 0,06 | 0,03 | 0,015 | 0,06 | 1 |
| Staph. aur. 133 | 0,25 | 0,125 | 0,06 | 0,03 | 0,015 |
| Streptococc.faecal. 9790 | 2 | 1 | 0,5 | 0,5 | 0,25 |
| Pseudomonas W. | 8 | 4 | 1 | 1 | 4 |

Agarverdünnungstest
Isosensitest-Medium
Denley Multipoint-Inokulator

**Patentansprüche**

1. 7-Amino-1-cyclopropyl-6,8-difluor-1,4-dihydro-4-oxo-chinolincarbonsäuren der Formel (I)

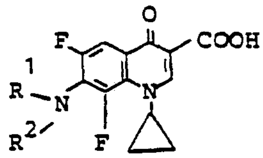

(I)

in welcher

$R^1$ und $R^2$ gleich oder verschieden sein können und für einen gegebenenfalls durch eine Hydroxy-, Amino-, Methylamino- oder Dimethylaminogruppe substituierten $C_1$-$C_4$-Alkylrest stehen und weiterhin gemeinsam mit dem Stickstoffatom an das sie gebunden sind, einen 5- oder 6-gliedrigen heterocyclischen Ring bilden können, der als Ringglied zusätzlich die Atome oder Gruppen —O—, —S—, —SO—, —$SO_2$— oder >N—$R^3$ enthalten kann und der gegebenenfalls an den Kohlenstoffatomen ein- bis dreifach durch $C_1$-$C_4$-Alkyl, Hydroxy, Alkoxy mit 1 bis 3 Kohlenstoffatomen, Amino, Methylamino oder Ethylamino substituiert sein kann, wobei jeweils ein Kohlenstoffatom nur einen Substituenten tragen kann, wobei

$R^3$ für Wasserstoff, eine verzweigte oder unverzweigte Alkyl-, Alkenyl- oder Alkinylgruppe mit 1 bis 6 Kohlenstoffatomen, die gegebenenfalls durch eine Hydroxy-, Trifluormethylmercapto-, Alkoxy-, Alkylmercapto-, Alkylamino- oder Dialkylamino-gruppe mit 1 bis 3 Kohlenstoffatomen für einen Alkylrest, die Cyanogruppe, die Alkoxycarbonylgruppe mit 1 bis 4 Kohlenstoffatomen im Alkoholteil substituiert sein kann, die Benzyloxycarbonylgruppe, eine gegebenenfalls im Phenylrest durch Nitro oder Amino substituierte Phenylalkylgruppe mit bis zu 4 Kohlenstoffatomen im aliphatischen Teil, einen gegebenenfalls durch Hydroxy, Methoxy, Chlor und Fluor ein- oder zweifach substituierten Phenylrest, einen gegebenenfalls durch Hydroxy, Methoxy, Chlor und Fluor ein- oder zweifach substituierten Phenacylrest, einen Oxoalkylrest mit bis zu 6 Kohlenstoffatomen sowie einen Cycloalkyl-alkylrest mit bis zu 6 Kohlenstoffatomen im cyclischen Teil und bis zu 3 Kohlenstoffatomen im acyclischen Teil steht, ferner einen Rest $COR_4$, CN oder $SO_2R^5$ bedeutet, wobei

$R^4$ Wasserstoff, gegebenenfalls durch 1 oder 2 Substituenten aus der Reihe Amino, Alkoxycarbonyl mit 1 bis 3 Kohlenstoffatomen im Alkylteil, Carboxy, Alkoxy mit 1 bis 3 Kohlenstoffatomen oder Trifluormethylthio substituiertes geradkettiges oder verzweigtes Alkyl mit 1 bis 4 C-Atomen, gegebenenfalls durch Chlor, Hydroxy, Amino oder Carboxy substituiertes Phenyl, Alkoxy mit 1 bis 4 C-Atomen, Alkylthio mit 1 bis 2 C-Atomen, Benzyloxy, Amino, gegebenenfalls durch Alkoxycarbonyl mit 1 bis 3 C-Atomen im Alkylteil oder Carboxy substituiertes Alkylamino mit 1 bis 5 C-Atomen,

$R^5$ geradkettiges oder verzweigtes Alkyl mit 1 bis 3 C-Atomen, Phenyl oder Methylphenyl darstellt, und deren pharmazeutisch verwendbaren Salze.

2. 7-Amino-1-cyclopropyl-6,8-difluor-1,4-dihydro-4-oxo-chinolincarbonsäuren der Formel (I)

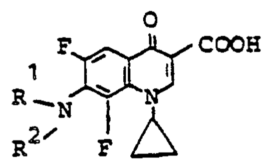

(I)

in welcher

$R^1$ und $R^2$ gleich oder verschieden sein können und für einen gegebenenfalls durch eine Hydroxy- oder Aminogruppe substituierten $C_1$-$C_3$-Alkylrest stehen und weiterhin gemeinsam mit dem Stickstoffatom, an das sie gebunden sind, einen 5- oder 6-gliedrigen heterocyclischen Ring bilden können, der als Ringglied zusätzlich die Atome oder Gruppen —O—, —S—, —$SO_2$— oder >N—$R^3$ enthalten kann und der gegebenenfalls an den Kohlenstoffatomen ein- bis zweifach durch $C_1$-$C_3$-Alkyl, Hydroxy, Amino oder Methylamino substituiert sein kann, wobei jeweils ein Kohlenstoffatom nur einen Substituenten tragen kann,

$R^3$ für Wasserstoff, eine verzweigte oder unverzweigte Alkyl-, Alkenyl- oder Alkinylgruppe mit 1 bis 4 Kohlenstoffatomen, die gegebenenfalls durch eine Hydroxy-, Trifluormethylmercapto-, Alkoxy- oder Alkylmercaptogruppe mit 1 bis 2 Kohlenstoffatomen für einen Alkylrest die Cyanogruppe, die Alkoxycarbonylgruppe mit 1 bis 3 Kohlenstoffatomen im Alkoholteil substituiert sein kann, die Benzyloxycarbonylgruppe, eine gegebenenfalls im Phenylrest durch Nitro oder Amino substituierte Phenylalkylgruppe mit bis zu 2 Kohlenstoffatomen im aliphatischen Teil, einen Phenacylrest, einen Oxoalkylrest mit bis zu 5 Kohlenstoffatomen sowie einen Cycloalkyl-alkylrest mit bis zu 6 Kohlenstoffatomen im cyclischen Teil und bis zu 2 Kohlenstoffatomen im acyclischen Teil steht, ferner einen Rest $COR^4$, CN oder $SO_2R^5$ bedeutet, wobei

$R^4$ Wasserstoff, gegebenenfalls durch 1 oder 2 Substituenten aus der Reihe Amino, Methoxycarbonyl, Carboxy, Alkoxy mit 1 bis 2 Kohlenstoffatomen oder Trifluormethylthio substituiertes geradkettiges oder

verzweigtes Alkyl mit 1 bis 3 C-Atomen, gegebenenfalls durch Chlor oder Hydroxy substituiertes Phenyl, Alkoxy, gegebenenfalls durch Alkoxycarbonyl mit 1 bis 2 C-Atomen im Alkylteil oder Carboxy substituiertes Alkylamino mit 1 bis 5 C-Atomen, und

$R^5$ Methyl, Ethyl, Phenyl oder Methylphenyl bedeutet.

3. 7-Amino-1-cyclopropyl-6,8-difluor-1,4-dihydro-4-oxo-chinolincarbonsäuren der Formel (I)

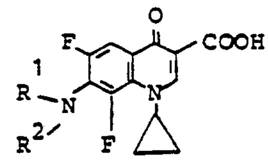

(I)

in welcher

$R^1$ und $R^2$ gleich oder verschieden sein können und für einen gegebenenfalls durch eine Hydroxygruppe substituierten $C_1$-$C_2$-Alkylrest stehen und weiterhin gemeinsam mit dem Stickstoffatom, an das sie gebunden sin, einen 6-gliedrigen heterocyclischen Ring bilden können, der als Ringglied zusätzlich die Gruppe $>N-R^3$ enthalten kann und der gegebenenfalls an den Kohlenstoffatomen ein- bis zweifach durch $C_1$-$C_2$-Alkyl oder Hydroxy substituiert sein kann, wobei jeweils ein Kohlenstoffatom nur einen Substituenten tragen kann,

wobei

$R^3$ für Wasserstoff, eine verzweigte oder unverzweigte Alkyl-, Alkenyl- oder Alkinylgruppe mit 1 bis 3 Kohlenstoffatomen, die gegebenenfalls durch eine Hydroxygruppe, die Alkoxycarbonylgruppe mit 1 bis 2 Kohlenstoffatomen im Alkoholteil substituiert sein kann, eine gegebenenfalls im Phenylrest durch Amino substituierte Benzylgruppe, einen Phenacylrest, einen Oxoalkylrest mit bis zu 4 Kohlenstoffatomen sowie einen Cyclopropylmethylrest steht, ferner einen Rest $COR^4$ oder $SO_2R^5$ bedeutet, wobei

$R^4$ Wasserstoff, gegebenenfalls durch einen Substituenten aus der Reihe Amino oder Carboxy substituiertes Alkyl mit 1 bis 2 C-Atomen, Alkoxy mit 1 bis 2 C-Atomen oder Benzyloxy und

$R^5$ Methyl bedeutet.

4. 1-Cyclopropyl-6,8-difluor-7-(1-piperazinyl)-1,4-dihydro-4-oxo-3-chinolincarbonsäure.

5. 1-Cyclopropyl-6,8-difluor-7-(4-methyl-1-piperazinyl)-1,4-dihydro-4-oxo-3-chinolincarbonsäure.

6. 1-Cyclopropyl-6,8-difluor-7-(1-pyrrolidinyl)-1,4-dihydro-4-oxo-3-chinolincarbonsäure.

7. 1-Cyclopropyl-6,7,8-trifluor-1,4-dihydro-4-oxo-3-chinolin-carbonsäure.

8. Verfahren zur Herstellung von 7-Amino-1-cyclopropyl-6,8-difluor-1,4-dihydro-4-oxo-chinolincarbonsäuren der Formel (I)

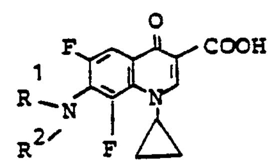

(I)

in welcher

$R^1$ und $R^2$ gleich oder verschieden sein können und für einen gegebenenfalls durch eine Hydroxy-, Amino-, Methylamino- oder Dimethylaminogruppe substituierten $C_1$-$C_4$-Alkylrest stehen und weiterhin gemeinsam mit dem Stickstoffatom an das sie gebunden sind, einen 5- oder 6-gliedrigen heterocyclischen Ring bilden können, der als Ringglied zusätzlich die Atome oder Gruppen —O—, —S—, —SO—, —$SO_2$— oder $>N-R^3$ enthalten kann und der gegebenenfalls an den Kohlenstoffatomen ein- bis dreifach durch $C_1$-$C_4$-Alkyl, Hydroxy, Alkoxy mit 1 bis 3 Kohlenstoffatomen, Amino, Methylamino oder Ethylamino substituiert sein kann, wobei jeweils ein Kohlenstoffatom nur einen Substituenten tragen kann,

wobei

$R^3$ für Wasserstoff, eine verzweigte oder unverzweigte Alkyl-, Alkenyl- oder Alkinylgruppe mit 1 bis 6 Kohlenstoffatomen, die gegebenenfalls durch eine Hydroxy-, Trifluormethylmercapto-, Alkoxy-, Alkylmercapto-, Alkylamino- oder Dialkylamino-gruppe mit 1 bis 3 Kohlenstoffatomen für einen Alkylrest, die Cyanogruppe, die Alkoxycarbonylgruppe mit 1 bis 4 Kohlenstoffatomen im Alkoholteil substituiert sein kann, die Benzyloxycarbonylgruppe, eine gegebenenfalls im Phenylrest durch Nitro oder Amino substituierte Phenylalkylgruppe mit bis zu 4 Kohlenstoffatomen im aliphatischen Teil, einen gegebenenfalls durch Hydroxy, Methoxy, Chlor und Fluor ein- oder zweifach substituierten Phenylrest, einen gegebenenfalls durch Hydroxy, Methoxy, Chlor und Fluor ein- oder zweifach substituierten Phenacylrest, einen Oxoalkylrest mit bis zu 6 Kohlenstoffatomen sowie einen Cycloalkyl-alkylrest mit bis zu 6 Kohlenstoffatomen im cyclischen Teil und bis zu 3 Kohlenstoffatomen im acyclischen Teil steht, ferner einen Rest $COR^4$, CN oder $SO_2R^5$ bedeutet, wobei

$R^4$ Wasserstoff, gegebenenfalls durch 1 oder 2 Substituenten aus der Reihe Amino, Alkoxycarbonyl mit 1 bis 3 Kohlenstoffatomen im Alkylteil, Carboxy, Alkoxy mit 1 bis 3 Kohlenstoffatomen oder Trifluormethylthio substituiertes geradkettiges oder verzweigtes Alkyl mit 1 bis 4 C-Atomen, gegebenenfalls durch Chlor, Hydroxy, Amino oder Carboxy substituiertes Phenyl, Alkoxy mit 1 bis 4 C-Atomen,

Alkylthio mit 1 bis 2 C-Atomen, Benzyloxy, Amino, gegebenenfalls durch Alkoxycarbonyl mit 1 bis 3 C-Atomen im Alkylteil oder Carboxy substituiertes Alkylamino mit 1 bis 5 C-Atomen,

$R^5$ geradkettiges oder verzweigtes Alkyl mit 1 bis 3 C-Atomen, Phenyl oder Methylphenyl darstellt, und deren pharmazeutisch verwendbaren Salze, dadurch gekennzeichnet, daß man entweder

a) Trifluor-chinolincarbonsäure der Formel (II)

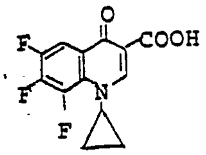

(II)

mit Aminen der Formel (III)

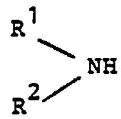

(III)

in welcher

$R^1$ und $R^2$ die oben angegebene Bedeutung haben, gegebenenfalls in Gegenwart von Säurebindern, umsetzt,

oder

b) eine 7-Piperazinylchinoloncarbonsäure der Formel (IV)

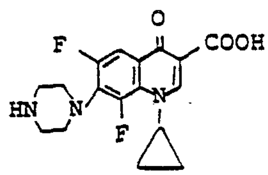

(IV)

in welcher der Piperazinylrest an den Kohlenstoffatomen 1- bis 3-fach durch $C_1$-$C_4$-Alkyl substituiert sein kann, wobei jeweils ein Kohlenstoffatom nur einen Substituenten tragen kann, mit Verbindungen der Formel (V)

$$R^3X \qquad (V)$$

in welcher

$R^3$ die oben angegebene Bedeutung hat, jedoch nicht Wasserstoff sein kann, und

X Fluor, Chlor, Brom, Iod, Acyloxy, Ethoxy, Phenoxy, 4-Nitrophenoxy bedeutet,

in Gegenwart von Säurebindern, umsetzt, oder

c) eine Piperazinyl-chinoloncarbonsäure der Formel (IV), in welcher der Piperazinylrest an den Kohlenstoffatomen 1- bis 3-fach durch $C_1$-$C_4$-Alkyl substituiert sein kann, wobei jeweils ein Kohlenstoffatom nur einen Substituenten tragen kann, mit Anhydriden der Formel (VI)

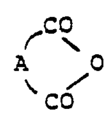

(VI)

in welcher A eine gegebenenfalls substituierte Alkylenkette mit 2 oder 3 Kohlenstoffatomen oder einen Arylenrest bedeutet, zu den Verbindungen der Formel (Ia)

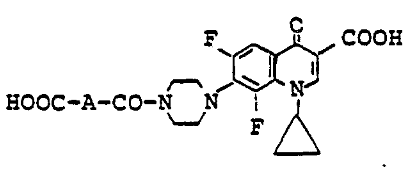

(Ia)

in welcher der Piperazinylrest an den Kohlenstoffatomen 1- bis 3-fach durch $C_1$-$C_4$-Alkyl substituiert sein kann, wobei jeweils ein Kohlenstoffatom nur einen Substituenten tragen kann, umsetzt, oder

d) eine Piperazinyl-chinoloncarbonsäure der Formel IV, in welcher der Piperazinylrest an den Kohlenstoffatomen 1 bis 3 fach durch $C_1$-$C_4$-Alkyl substituiert sein kann, wobei jeweils ein Kohlenstoffatom nur einen Substituenten tragen kann, mit Michael-Acceptoren der Formel (VII)

$$B{-}CH{=}CH_2 \qquad (VII)$$

in der B für CN, CO—$R^6$ oder $COOR^7$ steht, wobei
$R^6$ für Methyl oder Ethyl und
$R^7$ für Methyl, Ethyl, n- oder i-Propyl oder Benzyl steht, umsetzt.

9. Arzneimittel, gekennzeichnet durch einen Gehalt an mindestens einer 7-Amino-1-cyclopropyl-6,8-difluor-1,4-dihydro-4-oxo-3-chinolincarbonsäure gemäß Anspruch 1.

10. Verfahren zur Herstellung von 1-Cyclopropyl-6,7,8-trifluor-1,4-dihydro-4-oxo-3-chinolincarbonsäure der Formel (II)

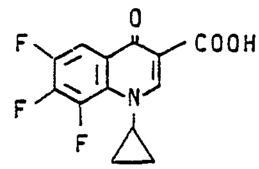
(II)

dadurch gekennzeichnet, daß man Malonsäurediethylester in Gegenwart von Magnesiumethylat mit 2,3,4,5-Tetrafluorbenzoylchlorid zum Aroylmalonester der Formel (3)

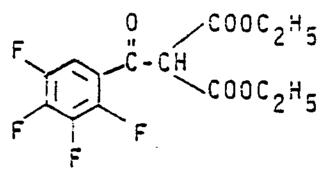
(3)

acyliert, den Aroylmalonester (3) in wäßrigem Medium mit katalytischen Mengen Schwefelsäure oder p-Toluolsulfonsäure partiell verseift und decarboxyliert zum Aroylessigsäureethylester der Formel (4)

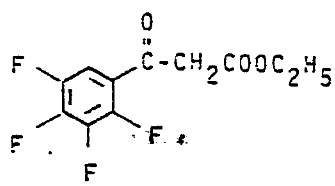
(4)

diesen mit o-Ameisensäure-triethylester/Acetanhydrid in den 2-(2,3,4,5-Tetrafluorbenzoyl)-3-ethoxy-acrylsäureethylester (5)

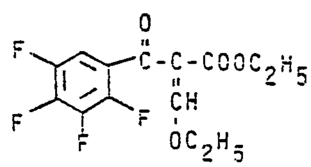
(5)

überführt, die Verbindung der Formel (5) mit Cyclopropylamin in einem Lösungsmittel wie Methylenchlorid, Alkohol, Chloroform, Cyclohexan oder Toluol in eine Verbindung der Formel (6)

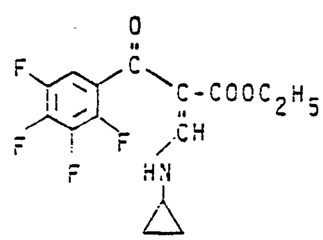
(6)

überführt, die Verbindung der Formel (6) bei Temperaturen von etwa 60 °C bis 300 °C in Dioxan, Dimethylsulfoxid, N-Methylpyrrolidon, Sulfolan, Hexamethylphosphorsäuretriamid oder Dimethylformamid in Gegenwart eines Säurebinders cyclisiert zur Verbindung der Formel (7)

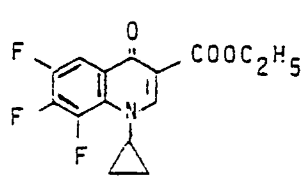
(7)

und gegebenenfalls die Verbindung der Formel (7) unter sauren oder basischen Bedingungen zur 1-Cyclopropyl-6,7,8-trifluor-1,4-dihydro-4-oxo-3-chinolincarbonsäure (II) hydrolysiert.

11. Verwendung von Verbindungen der allgemeinen Formel (I)

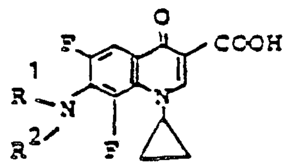

(I)

in welcher

$R^1$ und $R^2$ gleich oder verschieden sein können und für einen gegebenenfalls durch eine Hydroxy-, Amino-, Methylamino- oder Dimethylaminogruppe substituierten $C_1$-$C_4$-Alkylrest stehen und weiterhin gemeinsam mit dem Stickstoffatom an das sie gebunden sind, einen 5- oder 6-gliedrigen heterocyclischen Ring bilden können, der als Ringglied zusätzlich die Atome oder Gruppen —O—, —S—, —SO—, —$SO_2$— oder >N—$R^3$ enthalten kann und der gegebenenfalls an den Kohlenstoffatomen ein- bis dreifach durch $C_1$-$C_4$-Alkyl, Hydroxy, Alkoxy mit 1 bis 3 Kohlenstoffatomen, Amino, Methylamino oder Ethylamino substituiert sein kann, wobei jeweils ein Kohlenstoffatom nur einen Substituenten tragen kann, wobei

$R^3$ für Wasserstoff, eine verzweigte oder unverzweigte Alkyl-, Alkenyl- oder Alkinylgruppe mit 1 bis 6 Kohlenstoffatomen, die gegebenenfalls durch eine Hydroxy-, Trifluormethylmercapto-, Alkoxy-, Alkylmercapto-, Alkylamino- oder Dialkylamino-gruppe mit 1 bis 3 Kohlenstoffatomen für einen Alkylrest, die Cyanogruppe, die Alkoxycarbonylgruppe mit 1 bis 4 Kohlenstoffatomen im Alkoholteil substituiert sein kann, die Benzyloxycarbonylgruppe, eine gegebenenfalls im Phenylrest durch Nitro oder Amino substituierte Phenylalkylgruppe mit bis zu 4 Kohlenstoffatomen im aliphatischen Teil, einen gegebenenfalls durch Hydroxy, Methoxy, Chlor und Fluor ein- oder zweifach substituierten Phenylrest, einen gegebenenfalls durch Hydroxy, Methoxy, Chlor und Fluor ein- oder zweifach substituierten Phenacylrest, einen Oxoalkylrest mit bis zu 6 Kohlenstoffatomen sowie einen Cycloalkyl-alkylrest mit bis zu 6 Kohlenstoffatomen im cyclischen Teil und bis zu 3 Kohlenstoffatomen im acyclischen Teil steht, ferner einen Rest $COR^4$, CN oder $SO_2R^5$ bedeutet, wobei

$R^4$ Wasserstoff, gegebenenfalls durch 1 oder 2 Substituenten aus der Reihe Amino, Alkoxycarbonyl mit 1 bis 3 Kohlenstoffatomen im Alkylteil, Carboxy, Alkoxy mit 1 bis 3 Kohlenstoffatomen oder Trifluormethylthio substituiertes geradkettiges oder verzweigtes Alkyl mit 1 bis 4 C-Atomen, gegebenenfalls durch Chlor, Hydroxy, Amino oder Carboxy substituiertes Phenyl, Alkoxy mit 1 bis 4 C-Atomen, Alkylthio mit 1 bis 2 C-Atomen, Benzyloxy, Amino, gegebenenfalls durch Alkoxycarbonyl mit 1 bis 3 C-Atomen im Alkylteil oder Carboxy substituiertes Alkylamino mit 1 bis 5 C-Atomen,

$R^5$ geradkettiges oder verzweigtes Alkyl mit 1 bis 3 C-Atomen, Phenyl oder Methylphenyl darstellt, und deren pharmazeutisch verwendbaren Salze zur Herstellung von Arzneimitteln.

## Claims

1. 7-Amino-1-cyclopropyl-6,8-difluoro-1,4-dihydro-4-oxo-quinolinecxylic acids of the formula (I)

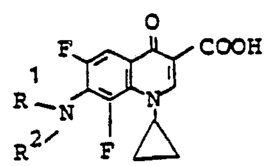

(I)

in which

$R^1$ and $R^2$ can be identical or different and represent a $C_1$-$C_4$-alkyl radical which is optionally substituted by a hydroxyl, amino, methylamino or dimethylamino group and furthermore, together with the nitrogen atom to which they are bonded, can form a 5- or 6-membered heterocyclic ring which can additionally contain, as a ring member, the atoms or groups —O—, —S—, —SO—, —$SO_2$— or >N—$R^3$ and which can optionally be mono- to trisubstituted at the carbon atoms by $C_1$-$C_4$-alkyl, hydroxyl, alkoxy with 1 to 3 carbon atoms, amino, methylamino or ethylamino, and each carbon atom concerned can carry only one substituent, wherein

$R^3$ represents hydrogen, a branched or unbranched alkyl, alkenyl or alkinyl group which has 1 to 6 carbon atoms and can optionally be substituted by a hydroxyl, trifluoromethylmercapto, alkoxy, alkylmercapto, alkylamino or dialkylamino group with 1 to 3 carbon atoms per alkyl radical, the cyano group or the alkoxycarbonyl group with 1 to 4 carbon atoms in the alcohol part, the benzyloxycarbonyl group, a phenylalkyl group which has up to 4 carbon atoms in the aliphatic part and is optionally substituted in the phenyl radical by nitro or amino, a phenyl radical which is optionally mono- or di-

substituted by hydroxyl, methoxy, chlorine and fluorine, a phenacyl radical which is optionally mono- or disubstituted by hydroxyl, methoxy, chlorine and fluorine, an oxoalkyl radical with up to 6 carbon atoms and a cycloalkyl-alkyl radical with up to 6 carbon atoms in the cyclic part and up to 3 carbon atoms in the acyclic part, and also denotes a radical $COR^4$, CN or $SO_2R^5$, wherein

$R^4$ represents hydrogen, straight-chain or branched alkyl which has 1 to 4 C atoms and is optionally substituted by 1 or 2 substituents from the series comprising amino, alkoxycarbonyl with 1 to 3 carbon atoms in the alkyl part, carboxyl, alkoxy with 1 to 3 carbon atoms or trifluoromethylthio, phenyl which is optionally substituted by chlorine, hydroxyl, amino or carboxyl, alkoxy with 1 to 4 C atoms, alkylthio with 1 to 2 C atoms, benzyloxy, amino, or alkylamino which has 1 to 5 C atoms and is optionally substituted by alkoxycarbonyl with 1 to 3 C atoms in the alkyl part or carboxyl, and

$R^5$ represents straight-chain or branched alkyl with 1 to 3 C atoms, phenyl or methylphenyl, and their pharmaceutically usable salts.

2. 7-Amino-1-cyclopropyl-6,8-difluoro-1,4-dihydro-4-oxo-quinolinecarboxylic acids of the formula (I)

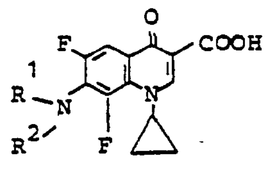

(I)

in which

$R^1$ and $R^2$ can be identical or different and represent a $C_1$-$C_3$-alkyl radical which is optionally substituted by a hydroxyl or amino group and furthermore, together with the nitrogen atom to which they are bonded, can form a 5- or 6-membered heterocyclic ring which can additionally contain, as a ring member, the atoms or groups —O—, —S—, —$SO_2$— or >N—$R^3$ and which can optionally be mono- to disubstituted at the carbon atoms by $C_1$-$C_3$-alkyl, hydroxyl, amino or methylamino, and each carbon atom concerned can carry only one substituent,

$R^3$ represents hydrogen, a branched or unbranched alkyl, alkenyl or alkinyl group which has 1 to 4 carbon atoms and can optionally be substituted by a hydroxyl, trifluoromethylmercapto, alkoxy or alkylmercapto group with 1 to 2 carbon atoms per alkyl radical, the cyano group or the alkoxycarbonyl group with 1 to 3 carbon atoms in the alcohol part, the benzyloxycarbonyl group, a phenylalkyl group which has up to 2 carbon atoms in the aliphatic part and is optionally substituted in the phenyl radical by nitro or amino, a phenacyl radical, an oxoalkyl radical with up to 5 carbon atoms and a cycloalkyl-alkyl radical which has up to 6 carbon atoms in the cyclic part and up to 2 carbon atoms in the acyclic part, and furthermore denotes a radical $COR^4$, CN or $SO_2R^5$, wherein

$R^4$ denotes hydrogen, straight-chain or branched alkyl which has 1 to 3 C atoms and is optionally substituted by 1 or 2 substituents from the series comprising amino, methoxycarbonyl, carbonyl, alkoxy with 1 to 2 carbon atoms or trifluoromethylthio, phenyl which is optionally substituted by chlorine or hydroxyl, alkoxy, or alkylamino which has 1 to 5 C atoms and is optionally substituted by alkoxycarbonyl with 1 to 2 C atoms in the alkyl part or carboxyl, and

$R^5$ denotes methyl, ethyl, phenyl or methylphenyl.

3. 7-Amino-1-cyclopropyl-6,8-difluoro-1,4-dihydro-4-oxo-quinolinecarboxylic acids of the formula (I)

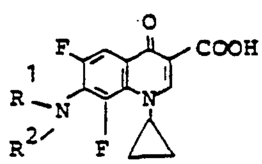

(I)

in which

$R^1$ and $R^2$ can be identical or different and represent a $C_1$-$C_2$-alkyl radical which is optionally substituted by a hydroxyl group and furthermore, together with the nitrogen atom to which they are bonded, can form a 6-membered heterocyclic ring which can additionally contain the group >N—$R^3$ as a ring member and which can optionally be mono- to disubstituted at the carbon atoms by $C_1$-$C_2$-alkyl or hydroxyl, and each carbon atom concerned can carry only one substituent, wherein

$R^3$ represents hydrogen, a branched or unbranched alkyl, alkenyl or alkinyl group which has 1 to 3 carbon atoms and can optionally be substituted by a hydroxyl group or the alkoxycarbonyl group with 1 to 2 carbon atoms in the alcohol part, a benzyl group which is optionally substituted in the phenyl radical by amino, a phenacyl radical, an oxoalkyl radical with up to 4 carbon atoms and a cyclopropylmethyl radical, and furthermore denotes a radical $COR^4$ or $SO_2R^5$, wherein

$R^4$ denotes hydrogen, alkyl which has 1 to 2 C atoms and is optionally substituted by a substituent from the series comprising amino or carboxyl, alkoxy with 1 to 2 C atoms or benzyloxy and

$R^5$ denotes methyl.

4. 1-Cyclopropyl-6,8-difluoro-7-(1-piperazinyl)-1,4-dihydro-4-oxo-3-quinolinecarboxylic acid.

5. 1-Cyclopropyl-6,8-difluoro-7-(4-methyl-1-piperazinyl)-1,4-dihydro-4-oxo-3-quinolinecarboxylic acid.

6. 1-Cyclopropyl-6,8-difluoro-7-(1-pyrrolidinyl)-1,4-dihydro-4-oxo-3-quinolinecarboxylic acid.

7. 1-Cyclopropyl-6,7,8-trifluoro-1,4-dihydro-4-oxo-3-quinoline-carboxylic acid.

8. Process for the preparation of 7-amino-1-cyclopropyl-6,8-difluoro-1,4-dihydro-4-oxo-quinolinecarboxylic acids of the formula (I)

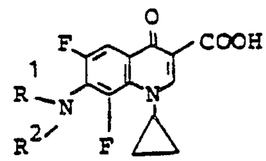

(I)

in which

$R^1$ and $R^2$ can be identical or different and represent a $C_1$-$C_4$-alkyl radical which is optionally substituted by a hydroxyl, amino, methylamino or dimethylamino group and furthermore, together with the nitrogen atom to which they are bonded, can form a 5- or 6-membered heterocyclic ring which can additionally contain, as a ring member, the atoms or groups —O—, —S—, —SO—, —$SO_2$— or >N—$R^3$ and which can optionally be mono- to trisubstituted at the carbon atoms by $C_1$-$C_4$-alkyl, hydroxyl, alkoxy with 1 to 3 carbon atoms, amino, methylamino or ethylamino, and each carbon atom concerned can carry only one substituent, wherein

$R^3$ represents hydrogen, a branched or unbranched alkyl, alkenyl or alkinyl group which has 1 to 6 carbon atoms and can optionally be substituted by a hydroxyl, trifluoromethylmercapto, alkoxy, alkylmercapto, alkylamino or dialkylamino group with 1 to 3 carbon atoms per alkyl radical, the cyano group or the alkoxycarbonyl group with 1 to 4 carbon atoms in the alcohol part, the benzyloxycarbonyl group, a phenylalkyl group which has up to 4 carbon atoms in the aliphatic part and is optionally substituted in the phenyl radical by nitro or amino, a phenyl radical which is optionally mono- or di-substituted by hydroxyl, methoxy, chlorine and fluorine, a phenacyl radical which is optionally mono- or disubstituted by hydroxyl, methoxy, chlorine and fluorine, an oxoalkyl radical with up to 6 carbon atoms and a cycloalkyl-alkyl radical with up to 6 carbon atoms in the cyclic part and up to 3 carbon atoms in the acyclic part, and also denotes a radical $COR^4$, CN or $SO_2R^5$, wherein

$R^4$ represents hydrogen, straight-chain or branched alkyl which has 1 to 4 C atoms and is optionally substituted by 1 or 2 substituents from the series comprising amino, alkoxycarbonyl with 1 to 3 carbon atoms in the alkyl part, carboxyl, alkoxy with 1 to 3 carbon atoms or trifluoromethylthio, phenyl which is optionally substituted by chlorine, hydroxyl, amino or carboxyl, alkoxy with 1 to 4 C atoms, alkylthio with 1 to 2 C atoms, benzyloxy, amino, or alkylamino which has 1 to 5 C atoms and is optionally substituted by alkoxycarbonyl with 1 to 3 C atoms in the alkyl part or carboxyl, and

$R^5$ represents straight-chain or branched alkyl with 1 to 3 C atoms, phenyl or methylphenyl, and their pharmaceutically usable salts, characterised in that either

a) trifluoro-quinolinecarboxylic acid of the formula (II)

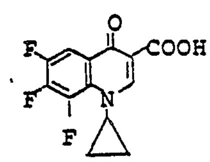

(II)

is reacted with amines of the formula (III)

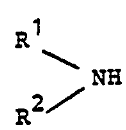

(III)

in which

$R^1$ and $R^2$ have the abovementioned meaning, if appropriate in the presence of acid-binding agents, or

b) a 7-piperazinylquinolonecarboxylic acid of the formula (IV)

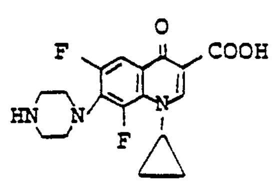

(IV)

in which the piperazinyl radical can be mono- to trisubstituted at the carbon atoms by $C_1$-$C_4$-alkyl, and each carbon atom concerned can carry only one substituent,
is reacted with compounds of the formula (V)

$R^3X$ (V)

in which
$R^3$ has the abovementioned meaning, but cannot be hydrogen, and
X denotes fluorine, chlorine, bromine, iodine, acyloxy, ethoxy, phenoxy or 4-nitrophenoxy,
in the presence of acid-binding agents, or

c) a piperazinyl-quinolonecarboxylic acid of the formula (IV) in which the piperazinyl radical can be mono- to trisubstituted at the carbon atoms by $C_1$-$C_4$-alkyl, and each carbon atom concerned can carry only one substituent, is reacted with anhydrides of the formula (VI)

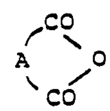

(VI)

in which A denotes an optionally substituted alkylene chain with 2 or 3 carbon atoms or an arylene radical, to give the compounds of the formula (Ia)

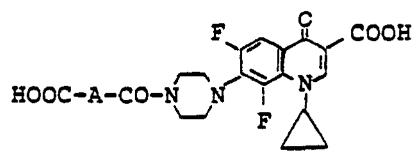

(Ia)

in which the piperazinyl radical can be mono- to trisubstituted at the carbon atoms by $C_1$-$C_4$-alkyl, and each carbon atom concerned can carry only one substituent, or

d) a piperazinyl-quinolonecarboxylic acid of the formula IV, in which the piperazinyl radical can be mono- to trisubstituted at the carbon atoms by $C_1$-$C_4$-alkyl, and each carbon atom concerned can carry only one substituent, is reacted with Michael acceptors of the formula (VII)

$B{-}CH{=}CH_2$ (VII)

in which B represents CN, CO—$R^6$ or $COOR^7$, wherein
$R^6$ represents methyl or ethyl and
$R^7$ represents methyl, ethyl, n- or i-propyl or benzyl.

9. Medicaments, characterised by a content of at least one 7-amino-1-cyclopropyl-6,8-difluoro-1,4-dihydro-4-oxo-3-quinolinecarboxylic acid according to Claim 1.

10. Process for the preparation of 1-cyclopropyl-6,7,8-trifluoro-1,4-dihydro-4-oxo-3-quinolinecarboxylic acid of the formula (II)

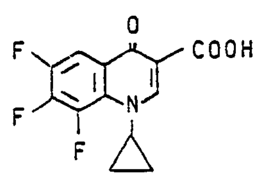

(II)

characterised in that diethyl malonate is acylated in the presence of magnesium methylate with 2,3,4,5-tetrafluorobenzoyl chloride to give the aroylmalonic ester of the formula (3)

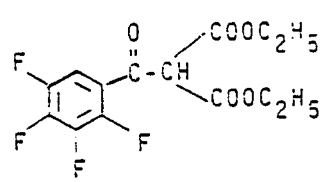

(3)

the aroylmalonic ester (3) is partially saponified and decarboxylated in an aqueous medium with catalytic quantitites of sulphuric acid or p-toluenesulphonic acid, to give ethyl aroylacetate of the formula (4)

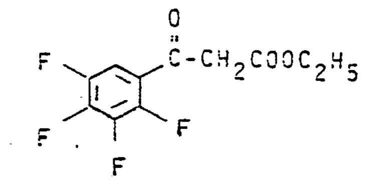

(4)

the latter is converted with triethyl o-formate/acetic anhydride into ethyl 2-(2,3,4,5-tetrafluorobenzoyl)-3-ethoxy-acrylate (5),

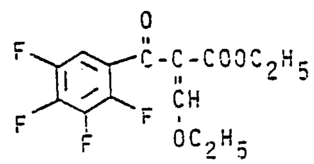

(5)

the compound of the formula (5) is converted with cyclopropylamine in a solvent such as methylene chloride, alcohol, chloroform, cyclohexane or toluene, into a compound of the formula (6)

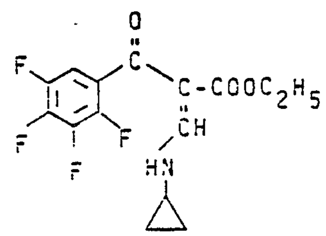

(6)

the compound of the formula (6) is cyclised, at temperatures of about 60 °C to 300 °C in dioxane, dimethyl sulphoxide, N-methyl-pyrrolidone, sulpholane, hexamethylphosphoric acid triamide or dimethyl formamide, in the presence of an acid-binding agent, to give the compound of the formula (7)

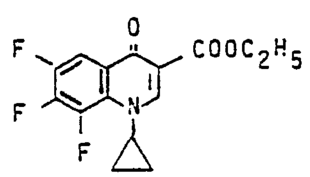

(7)

and, if desired, the compound of the formula (7) is hydrolysed, under acid or basic conditions to give 1-cyclopropyl-6,7,8-trifluoro-1,4-dihydro-4-oxo-3-quinolinecarboxylic acid (II).

11. Use of compounds of the general formula (I)

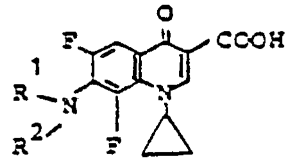

(I)

in which

$R^1$ and $R^2$ can be identical or different and represent a $C_1$-$C_4$-alkyl radical which is optionally substitued by a hydroxyl, amino, methylamino or dimethylamino group and furthermore, together with the nitrogen atom to which they are bonded, can form a 5- or 6-membered heterocyclic ring which can additionally contain, as a ring member, the atoms or groups —O—, —S—, —SO—, —$SO_2$— or $\geq$N—$R^3$ and which can optionally be mono- to trisubstituted at the carbon atoms by $C_1$-$C_4$-alkyl, hydroxyl, alkoxy with 1 to 3 carbon atoms, amino, methylamino or ethylamino, and each carbon atom concerned can carry only one substituent, wherein

$R^3$ represents hydrogen, a branched or unbranched alkyl, alkenyl or alkinyl group which has 1 to 6 carbon atoms and can optionally be substituted by a hydroxyl, trifluoromethylmercapto, alkoxy, alkylmercapto, alkylamino or dialkylamino group with 1 to 3 carbon atoms per alkyl radical, the cyano group or the alkoxycarbonyl group with 1 to 4 carbon atoms in the alcohol part, the benzyloxycarbonyl group, a phenylalkyl group which has up to 4 carbon atoms in the aliphatic part and is optionally substituted in the phenyl radical by nitro or amino, a phenyl radical which is optionally mono- or di-substituted by hydroxyl, methoxy, chlorine and fluorine, a phenacyl radical which is optionally mono- or

disubstituted by hydroxyl, methoxy, chlorine and fluorine, an oxoalkyl radical with up to 6 carbon atoms and a cycloalkyl-alkyl radical with up to 6 carbon atoms in the cyclic part and up to 3 carbon atoms in the acyclic part, and also denotes a radical $COR^4$, CN or $SO_2R^5$, wherein

$R^4$ represents hydrogen, straight-chain or branched alkyl which has 1 to 4 C atoms and is optionally substituted by 1 or 2 substituents from the series comprising amino, alkoxycarbonyl with 1 to 3 carbon atoms in the alkyl part, carboyxl, alkoxy with 1 to 3 carbon atoms or trifluoromethylthio, phenyl which is optionally substituted by chlorine, hydroxyl, amino or carboxyl, alkoxy with 1 to 4 C atoms, alkylthio with 1 to 2 C atoms, benzyloxy, amino, or alkylamino which has 1 to 5 C atoms and is optionally substituted by alkoxycarbonyl with 1 to 3 C atoms in the alkyl part or carboxyl, and

$R^5$ represents straight-chain or branched alkyl with 1 to 3 C atoms, phenyl or methylphenyl, and their pharmaceutically usable salts, for the preparation of medicaments.

**Revendications**

1. Acides 7-amino-1-cyclopropyl-6,8-difluoro-1,4-dihydro-4-oxo-quinoléine-carboxyliques de formule I

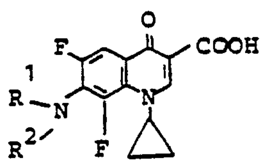

(I)

dans laquelle

$R^1$ et $R^2$, qui peuvent avoir des significations identiques ou différentes, représentent chacun un groupe alkyle en $C_1$-$C_4$ éventuellement substitué par un groupe hydroxy, amino, méthylamino ou diméthylamino, ou bien forment ensemble et avec l'atome d'azote auquel ils sont reliés un noyau hétérocyclique à 5 ou 6 chaînons contenant en outre, en tant que chaînons cycliques, les atomes ou groupes —O—, —S—, —SO—, —$SO_2$— ou $>$N—$R^3$ et qui peut le cas échéant être mono- à trisubstitué sur les atomes de carbone par des groupes alkyle en $C_1$-$C_4$, hydroxy, alcoxy en $C_1$-$C_3$, amino, méthylamino ou éthylamino, chaque atome de carbone ne pouvant porter qu'un seul substituant,

$R^3$ représente l'hydrogène ou un groupe alkyle, alcényle ou alcynyle droit ou ramifié en $C_1$-$C_6$ qui peut être le cas échéant substitué par un groupe hydroxy, trifluorométhylmercapto, alcoxy, alkylmercapto, alkylamino ou dialkylamino contenant 1 à 3 atomes de carbone par groupe alkyle, le groupe cyano, le groupe alcoxycarbonyle contenant 1 à 4 atomes de carbone dans la partie alcoolique, le groupe benzyloxycarbonyle, un groupe phénylalkyle contenant jusqu'à 4 atomes de carbone dans la partie aliphatique, éventuellement substitué dans la partie phényle par des groupes nitro ou amino, un groupe phényle éventuellement mono- ou di-substitué par des groupes hydroxy, méthoxy, le chlore et le fluor, un groupe phénacyle éventuellement mono- ou di-substitué par des groupes hydroxy, méthoxy, le chlore et le fluor, un groupe oxoalkyle contenant jusqu'à 6 atomes de carbone ou un groupe cycloalkyl-alkyle contenant jusqu'à 6 atomes de carbone dans la partie cyclique et jusqu'à 3 atomes de carbone dans la partie acyclique, ou un reste $COR^4$, CN ou $SO_2R^5$,

$R^4$ représente l'hydrogène, un groupe alkyle à chaîne droite ou ramifiée en $C_1$-$C_4$ portant éventuellement 1 ou 2 substituants choisis parmi les groupes amino, alcoxycarbonyle contenant 1 à 3 atomes de carbone dans la partie alkyle, carboxy, alcoxy en $C_1$-$C_3$ ou trifluorométhylthio, un groupe phényle éventuellement substitué par le chlore, des groupes hydroxy, amino ou carboxy, un groupe alcoxy en $C_1$-$C_4$, alkylthio en $C_1$-$C_2$, benzyloxy, amino, alkylamino en $C_1$-$C_5$ éventuellement substitué par un groupe alcoxycarbonyle contenant 1 à 3 atomes de carbone dans la partie alkyle ou par un groupe carboxy,

$R^5$ représente un groupe alkyle à chaîne droite ou ramifiée en $C_1$-$C_3$, phényle ou méthylphényle, et leurs sels acceptables pour l'usage pharmaceutique.

2. Acides 7-amino-1-cyclopropyl-6,8-difluoro-1,4-dihydro-4-oxo-quinoléine-carboxyliques de formule I

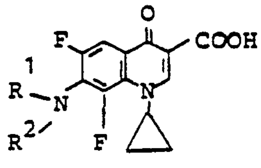

(I)

dans laquelle

$R^1$ et $R^2$, qui peuvent avoir des significations identiques ou différentes, représentent chacun un groupe alkyle en $C_1$-$C_3$ éventuellement substitué par un groupe hydroxy ou amino ou forment ensemble et avec l'atome d'azote auquel ils sont reliés un noyau hétérocyclique à 5 ou 6 chaînons contenant en

outre, en tant que chaînons cycliques, les atomes ou groupes —O—, —S—, —$SO_2$— ou >N—$R^3$ et qui peut le cas échéant être mono- ou di-substitué sur les atomes de carbone par des groupes alkyle en $C_1$-$C_3$, hydroxy, amino ou méthylamino, chaque atome de carbone ne pouvant porter qu'un seul substituant,

$R^3$ représente l'hydrogène, un groupe alkyle, alcényle ou alcynyle à chaîne droite ou ramifiée en $C_1$-$C_4$, qui peut le cas échéant être substitué par un groupe hydroxy, trifluorométhylmercapto, alcoxy ou alkylmercapto contenant 1 ou 2 atomes de carbone par groupe alkyle, le groupe cyano, le groupe alcoxycarbonyle contenant 1 à 3 atomes de carbone dans la partie alcoolique, le groupe benzyloxycarbonyle, un groupe phénylalkyle contenant jusqu'à 2 atomes de carbone dans la partie aliphatique, éventuellement substitué dans la partie phényle par des groupes nitro ou amino, un radical phénacyle, un groupe oxoalkyle contenant jusqu'à 5 atomes de carbone ou un groupe cycloalkyl-alkyle contenant jusqu'à 6 atomes de carbone dans la partie cyclique et jusqu'à 2 atomes de carbone dans la partie acyclique, ou encore un reste $COR_4$, CN ou $SO_2R^5$,

$R^4$ représente l'hydrogène, un groupe alkyle à chaîne droite ou ramifiée en $C_1$-$C_3$ portant éventuellement 1 ou 2 substituants choisis parmi les groupes amino, méthoxycarbonyle, carboxy, alcoxy en $C_1$-$C_2$ ou trifluorométhylthio, un groupe phényle éventuellement substitué par le chlore ou un groupe hydroxy, un groupe alcoxy, un groupe alkylamino en $C_1$-$C_5$ éventuellement substitué par un groupe alcoxycarbonyle contenant 1 à 2 atomes de carbone dans la partie alkyle ou par un groupe carboxy, et

$R^5$ représente un groupe méthyle, éthyle, phényle ou méthylphényle.

3. Acides 7-amino-1-cyclopropyl-6,8-difluoro-1,4-dihydro-4-oxo-quinoléine-carboxyliques de formule I

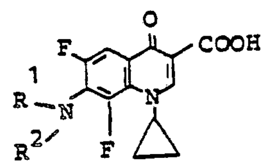

(I)

dans laquelle

$R^1$ et $R^2$, qui peuvent avoir des significations identiques ou différentes, représentent chacun un groupe alkyle en $C_1$-$C_2$ éventuellement substitué par un groupe hydroxy ou forment ensemble et avec l'atome d'azote auquel ils sont reliés un noyau hétérocyclique à 6 chaînons qui peut contenir en outre en tant que chaînon cyclique le groupe >N—$R^3$ et qui peut le cas échéant être mono- ou di-substitué sur les atomes de carbone par des groupes alkyle en $C_1$-$C_2$ ou hydroxy, chaque atome de carbone ne pouvant porter qu'un seul substituant,

$R^3$ représente l'hydrogène, un groupe alkyle, alcényle ou alcynyle à chaîne droite ou ramifiée en $C_1$-$C_3$ qui peut le cas échéant être substitué par un groupe hydroxy, le groupe alcoxycarbonyle contenant 1 à 2 atomes de carbone dans la partie alcoolique, un groupe benzyle éventuellement substitué dans la partie phényle par un groupe amino, un radical phénacyle, un groupe oxoalkyle contenant jusqu'à 4 atomes de carbone ou un groupe cyclopropylméthyle, ou encore un reste $COR^4$ ou $SO_2R^5$,

$R^4$ représente l'hydrogène, un groupe alkyle en $C_1$-$C_2$, alcoxy en $C_1$-$C_2$ ou benzyloxy portant éventuellement un substituant choisi parmi les groupes amino ou carboxy, et

$R^5$ représente un groupe méthyle.

4. L'acide 1-cyclopropyl-6,8-difluoro-7-(1-pipérazynyl)-1,4-dihydro-4-oxo-3-quinoléine-carboxylique.

5. L'acide 1-cyclopropyl-6,8-difluoro-7-(4-méthyl-1-pipérazinyl)-1,4-dihydro-4-oxo-3-quinoléine-carboxylique.

6. L'acide 1-cyclopropyl-6,8-difluoro-7-(1-pyrrolidinyl)-1,4-dihydro-4-oxo-3-quinoléine-carboxylique.

7. L'acide 1-cyclopropyl-6,7,8-trifluoro-1,4-dihydro-4-oxo-3-quinoléine-carboxylique.

8. Procédé de préparation des acides 7-amino-1-cyclo-propyl-6,8-difluoro-1,4-dihydro-4-oxo-quinoléine-carboxyliques de formule I

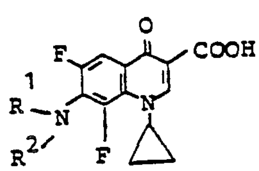

(I)

dans laquelle

$R^1$ et $R^2$, qui peuvent avoir des significations identiques ou différentes, représentent chacun un groupe alkyle en $C_1$-$C_4$ éventuellement substitué par un groupe hydroxy, amino, méthylamino ou diméthylamino, ou bien forment ensemble et avec l'atome d'azote auquel ils sont reliés un noyau hétérocyclique à 5 ou 6 chaînons contenant en outre, en tant que chaînons cycliques, les atomes ou groupes —O—, —S—, —SO—, $SO_2$— ou >N—$R^3$ et qui peut le cas échéant être mono- à trisubstitué sur les atomes de carbone par des groupes alkyle en $C_1$-$C_4$, hydroxy, alcoxy en $C_1$-$C_3$, amino, méthylamino ou éthylamino, chaque atome de carbone ne pouvant porter qu'un seul substituant,

$R^3$ représente l'hydrogène ou un groupe alkyle, alcényle ou alcynyle droit ou ramifié en $C_1$-$C_6$ qui

peut être le cas échéant substitué par un groupe hydroxy, trifluorométhylmercapto, alcoxy, alkylmercapto, alkylamino ou dialkylamino contenant 1 à 3 atomes de carbone par groupe alkyle, le groupe cyano, le groupe alcoxycarbonyle contenant 1 à 4 atomes de carbone dans la partie alcoolique, le groupe benzyloxycarbonyle, un groupe phénylalkyle contenant jusqu'à 4 atomes de carbone dans la partie aliphatique, éventuellement substitué dans la partie phényle par des groupes nitro ou amino, un groupe phényle éventuellement mono- ou di-substitué par des groupes hydroxy, méthoxy, le chlore et le fluor, un groupe phénacyle éventuellement mono- ou di-substitué par des groupes hydroxy, méthoxy, le chlore et le fluor, un groupe oxoalkyle contenant jusqu'à 6 atomes de carbone ou un groupe cycloalkyl-alkyle contenant jusqu'à 6 atomes de carbone dans la partie cyclique et jusqu'à 3 atomes de carbone dans la partie acyclique ou un reste $COR^4$, CN ou $SO_2R^5$,

$R^4$ représente l'hydrogène, un groupe alkyle à chaîne droite ou ramifiée en $C_1$-$C_4$ portant éventuellement 1 à 2 substituants choisis parmi les groupes amino, alcoxycarbonyle contenant 1 à 3 atomes de carbone dans la partie alkyle, carboxy, alcoxy en $C_1$-$C_3$ ou trifluorométhylthio, un groupe phényle éventuellement substitué par le chlore, des groupes hydroxy, amino ou carboxy, un groupe alcoxy en $C_1$-$C_4$, alkylthio en $C_1$-$C_2$, benzyloxy, amino, alkylamino en $C_1$-$C_5$ éventuellement substitué par un groupe alcoxycarbonyle contenant 1 à 3 atomes de carbone dans la partie alkyle ou par un groupe carboxy,

$R^5$ représente un groupe alkyle à chaîne droite ou ramifiée en $C_1$-$C_3$, phényle ou méthylphényle, et de leurs sels acceptables pour l'usage pharmaceutique, caractérisé en ce que :

a) ou bien on fait réagir l'acide trifluoro-quinoléine-carboxylique de formule II

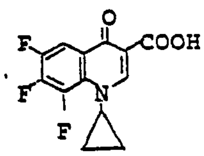

(II)

avec des amines de formule III

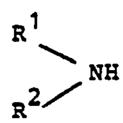

(III)

dans laquelle $R^1$ et $R^2$ ont les significations indiquées ci-dessus, éventuellement en présence d'accepteurs d'acides,

b) ou bien on fait réagir un acide 7-pipérazinyl-quinolone-carboxylique de formule IV

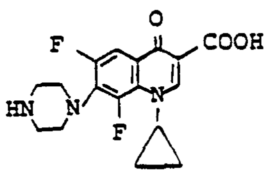

(IV)

dans laquelle le groupe pipérazinyle peut être mono à tri-substitué sur les atomes de carbone par des groupes alkyle en $C_1$-$C_4$, chaque atome de carbone ne pouvant porter qu'un seul substituant, avec des composés de formule V

$$R^3X \quad (V)$$

dans laquelle

$R^3$ a les significations indiquées ci-dessus à l'exception de l'hydrogène, et

X représente le fluor, le chlore, le brome, l'iode, un groupe alcoxy, éthoxy, phénoxy, 4-nitrophénoxy, en présence d'accepteurs d'acides,

c) ou bien on fait réagir un acide pipérazinyl-quinolone-carboxylique de formule IV dans lequel le groupe pipérazinyle peut être mono- à tri-substitué sur les atomes de carbone par des groupes alkyle en $C_1$-$C_4$, chaque atome de carbone ne pouvant porter qu'un seul substituant, avec des anhydrides de formule VI

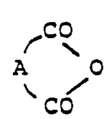

(VI)

dans laquelle A représente une chaîne alkylène en $C_2$ ou $C_3$ éventuellement substituée ou un radical arylène, ce qui donne les composés de formule Ia

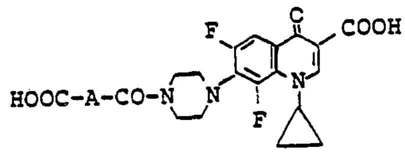

(Ia)

dans lesquels le groupe pipérazinyle peut être mono- à tri-substitué sur les atomes de carbone par des groupes alkyle en $C_1$-$C_4$, chaque atome de carbone ne pouvant porter qu'un seul substituant,

d) ou bien on fait réagir un acide pipérazinyl-quinolone-carboxylique de formule IV dans lequel le groupe pipérazinyle peut être mono- à tri-substitué sur les atomes de carbone par des groupes alkyle en $C_1$-$C_4$, chaque atome de carbone ne pouvant porter qu'un seul substituant, avec des accepteurs de Michael de formule VII

$$B—CH=CH_2 \quad \text{(VII)}$$

dans laquelle

B représente CN, CO—$R^6$ ou $COOR^7$,

$R^6$ représente un groupe méthyle ou éthyle, et

$R^7$ représente un groupe méthyle, éthyle, n- ou iso-propyle ou benzyle.

9. Médicament caractérisé en ce qu'il contient au moins un acide 7-amino-1-cyclopropyl-6,8-difluoro-1,4-dihydro-4-oxo-3-quinoléine-carboxylique selon la revendication 1.

10. Procédé de préparation de l'acide 1-cyclopropyl-6,7,8-trifluoro-1,4-dihydro-4-oxo-3-quinoléine-carboxylique de formule II

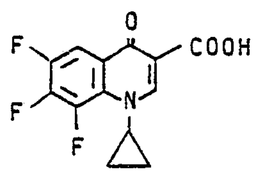

(II)

caractérisé en ce que l'on acyle le malonate de diéthyle en présence d'éthylate de magnésium à l'aide du chlorure de 2,3,4,5-tétrafluorobenzoyle, ce qui donne l'ester aroylmalonique de formule (3)

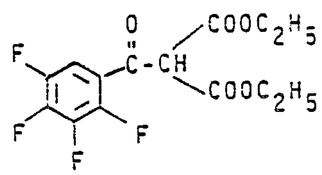

(3)

qu'on soumet à saponification partielle et décarboxylation en milieu aqueux à l'aide de quantités catalytiques d'acide sulfurique ou d'acide p-toluène-sulfonique, ce qui donne l'aroylacétate d'éthyle de formule (4)

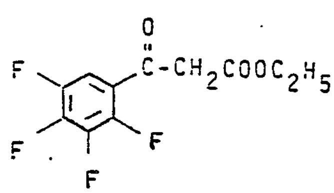

(4)

qu'on convertit à l'aide de l'orthoformiate de triéthyle/anhydride acétique en le 2-(2,3,4,5-tétrafluorobenzoyl)-3-éthoxy-acrylate d'éthyle (5).

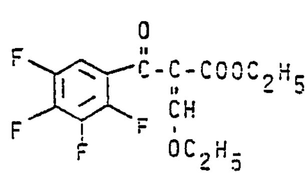

(5)

qu'on convertit à l'aide de la cyclopropylamine dans un solvant tel que le chlorure de méthylène, l'alcool, le chloroforme, le cyclohexane ou le toluène en un composé de formule (6)

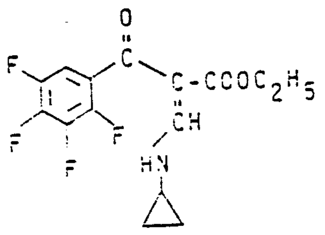

(6)

qu'on cyclise à des températures de 60 à 300 °C environ dans le dioxanne, le diméthylsulfoxyde, la N-méthylpyrrolidone, le sulfolan, l'hexaméthylphosphorotriamide ou le diméthylformamide en présence d'un accepteur d'acide, en un composé de formule VII

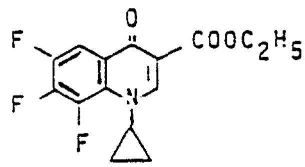

(7)

qu'on hydrolyse le cas échéant en milieu acide ou basique, en l'acide 1-cyclopropyl-6,7,8-trifluoro-1,4-dihydro-4-oxo-3-quinoléine-carboxylique II.

11. Utilisation des composés de formule générale I

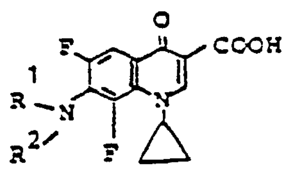

(I)

dans laquelle

$R^1$ et $R^2$, qui peuvent avoir des significations identiques ou différentes, représentent chacun un groupe alkyle en $C_1$-$C_4$ éventuellement substitué par un groupe hydroxy, amino, méthylamino ou diméthylamino ou forment ensemble et avec l'atome d'azote auquel ils sont reliés un hétérocycle à 5 ou 6 chaînons qui peut contenir en outre en tant que chaînons cycliques les atomes ou groupes —O—, —S—, —SO—, —$SO_2$— ou >N—$R^3$ et qui peut le cas échéant être mono- à tri-substitué sur les atomes de carbone par des groupes alkyle en $C_1$-$C_4$, hydroxy, alcoxy en $C_1$-$C_3$, amino, méthylamino ou éthylamino, chaque atome de carbone ne pouvant porter qu'un seul substituant,

$R^3$ représente l'hydrogène, un groupe alkyle, alcényle ou alcynyle droit ou ramifié en $C_1$-$C_6$, qui peut le cas échéant être substitué par un groupe hydroxy, trifluorométhylmercapto, alcoxy, alkylmercapto, alkylamino ou dialkylamino contenant 1 à 3 atomes de carbone dans chaque groupe alkyle, le groupe cyano, un groupe alcoxycarbonyle contenant 1 à 4 atomes de carbone dans la partie alcoolique, le groupe benzyloxycarbonyle, un groupe phénylalkyle contenant jusqu'à 4 atomes de carbone dans la partie aliphatique et éventuellement substitué dans le noyau phényle par des groupes nitro ou amino, un groupe phényle éventuellement mono- ou di-substitué par des groupes hydroxy, méthoxy, le chlore et le fluor, un radical phénacyle éventuellement mono- ou di-substitué par des groupes hydroxy, méthoxy, le chlore et le fluor, un groupe oxoalkyle contenant jusqu'à 6 atomes de carbone ou un groupe cycloalkyl-alkyle contenant jusqu'à 6 atomes de carbone dans la partie cyclique et jusqu'à 3 atomes de carbone dans la partie acyclique, ou un reste $COR^4$, CN ou $SO_2R^5$,

$R^4$ représente l'hydrogène, un groupe alkyle à chaîne droite ou ramifiée en $C_1$-$C_4$ portant le cas échéant 1 ou 2 substituants choisis parmi les groupes amino, alcoxycarbonyle contenant 1 à 3 atomes de carbone dans la partie alkyle, carboxy, alcoxy en $C_1$-$C_3$ ou trifluorométhylthio, un groupe phényle éventuellement substitué par le chlore, des groupes hydroxy, amino ou carboxy, un groupe alcoxy en $C_1$-$C_4$, alkylthio en $C_1$-$C_2$, benzyloxy, amino, alkylamino en $C_1$-$C_5$ éventuellement substitué par un groupe alcoxycarbonyle contenant 1 à 3 atomes de carbone dans la partie alkyle ou par un groupe carboxy,

$R^5$ représente un groupe alkyle à chaîne droite ou ramifiée en $C_1$-$C_3$, phényle ou méthylphényle, et de leurs sels acceptables pour l'usage pharmaceutique, pour la préparation de médicaments.